# FASCICULE DE BREVET EUROPEEN

(11) **EP 2 334 782 B1**
(45) Date de publication et mention de la délivrance du brevet: **09.07.2014**
(21) Numéro de dépôt: 09809346.1
(22) Date de dépôt: 28.08.2009
(51) Int. Cl.: C12N 1/16, C12N 1/20, A23L 1/30, A61K 35/74, C12R 1/225, C12R 1/85, A61K 36/064

(54) **MICROORGANISMES NON PHOTOSYNTHÉTIQUES ENRICHIS EN SÉLÉNIUM ORGANIQUE A PARTIR DE COMPOSÉS SÉLÉNO-HYDROXYACIDES ET LEURS APPLICATIONS EN NUTRITION ET COSMÉTIQUE**
MIT ORGANISCHEM SELEN AUS SELENOHYDROXYSÄURE-VERBINDUNGEN ANGEREICHERTE NICHT PHOTOSYNTHETISCHE MIKROORGANISMEN UND ANWENDUNGEN DAVON AUF DEN GEBIETEN ERNÄHRUNG UND KOSMETIK
NON-PHOTOSYNTHETIC MICRO-ORGANISMS ENRICHED WITH ORGANIC SELENIUM FROM SELENO-HYDROXYACID COMPOUNDS, AND APPLICATIONS THEREOF IN THE FIELDS OF NUTRITION AND COSMETICS

(30) Priorité: 29.08.2008 FR 0855825
(43) Date de publication de la demande: 22.06.2011
(73) Titulaire: Tetrahedron, 94300 Vincennes (FR)
(72) Inventeur: YADAN, Jean-Claude, F-93100 Montreuil Sous Bois (FR); MOUTET, Marc, F-94230 Cachan (FR)
(74) Mandataire: Regimbeau
(86) Numéro de dépôt international: PCT/EP2009/061165
(87) Numéro de publication internationale: WO 2010/023291

(56) Documents cités:
- EP-A- 1 602 716
- WO-A-03/078605
- WO-A-2006/008190
- US-B1- 6 197 295
- XIA SHU KAI ET AL: "Enriched selenium and its effects on growth and biochemical composition in Lactobacillus bulgaricus." JOURNAL OF AGRICULTURAL AND FOOD CHEMISTRY 21 MAR 2007, vol. 55, no. 6, 21 mars 2007 (2007-03-21), pages 2413-2417, XP002528578 ISSN: 0021-8561
- ANDREONI V ET AL: "SELENITE TOLERANCE AND ACCUMULATION IN THE LACTOBACILLUS SPECIES" ANNALS OF MICROBIOLOGY, DISTAM, MILAN, IT, vol. 50, no. 1, 1 janvier 2000 (2000-01-01), pages 77-88, XP008036008 ISSN: 1590-4261
- CALOMME M ET AL: "Seleno-lactobacillus: An organic selenium source" BIOLOGICAL TRACE ELEMENT RESEARCH, HUMANA PRESS, CLIFTON, NJ, US, vol. 47, no. 1-3, 1 janvier 1995 (1995-01-01), pages 379-383, XP009114178 ISSN: 0163-4984
- INFANTE HEIDI GOENAGA ET AL: "Selenium speciation analysis of selenium-enriched supplements by HPLC with ultrasonic nebulisation ICP-MS and electrospray MS/MS detection" JOURNAL OF ANALYTICAL ATOMIC SPECTROMETRY, vol. 19, no. 12, 2004, pages 1529-1538, XP002528577 ISSN: 0267-9477
- MCSHEEHY S ET AL: "Determination of Methionine and Selenomethionine in Selenium-Enriched Yeast by Species-Specific Isotope Dilution with Liquid Chromatography-Mass Spectrometry and Inductively Coupled Plasma Mass Spectrometry Detection" ANALYTICAL CHEMISTRY,, vol. 77, no. 1, 1 janvier 2005 (2005-01-01), pages 344-349, XP003023081
- AVOSCAN LAURE ET AL: "Seleno-L-methionine is the predominant organic form of selenium in Cupriavidus metallidurans CH34 exposed to selenite or selenate." APPLIED AND ENVIRONMENTAL MICROBIOLOGY SEP 2006, vol. 72, no. 9, septembre 2006 (2006-09), pages 6414-6416, XP002528579 ISSN: 0099-2240
- ALZATE A ET AL: "Comparison of biotransformation of inorganic selenium by Lactobacillus and Saccharomyces in lactic fermentation process of yogurt and kefir." JOURNAL OF AGRICULTURAL AND FOOD CHEMISTRY 24 SEP 2008, vol. 56, no. 18, 27 août 2008 (2008-08-27), pages 8728-8736, XP002528580 ISSN: 1520-5118
- EMMIE DUMONT ET AL: "Selenium speciation from food source to metabolites: a critical review" ANALYTICAL AND BIOANALYTICAL CHEMISTRY, SPRINGER, BERLIN, DE, vol. 385, no. 7, 8 juillet 2006 (2006-07-08), pages 1304-1323, XP019420329 ISSN: 1618-2650

## Description

L'invention se rapporte à l'enrichissement de microorganismes non photosynthétiques en sélénium organique, notamment à l'aide de composés de type séléno-hydroxyacides, et plus particulièrement à l'aide d'acide 2-hydroxy-4-méthylséléno-butanoïque, sous forme (D, L), ou d'un énantiomère, d'un sel, d'un dérivé ester ou amide de ce composé, ainsi que l'utilisation des microorganismes ainsi enrichis en nutrition animale ou humaine, en cosmétique ou en pharmacie.

Le sélénium est un micro-nutriment essentiel pour l'Homme et les mammifères notamment *(*Wendel, A. ; Phosphorus, Sulfur Silicon Relat Elem. ; 1992; 67, 1-4, 405-415*).* En particulier, il participe, sous la forme de L(+)-sélénocystéine ou de L(+)-sélénométhionine *(*Muller, S. et al. ; Arch. Microbiol., 1997; 168 ; 421*)* à la biosynthèse des sélénoprotéines telles que la Glutathion peroxydase, la Thiorédoxine réductase et la Sélénoprotéine **P.**

Chez l'Homme, des déficiences en sélénium ont été reportées, notamment dans le cas de patients soumis à une alimentation par voie parentérale durant de longues périodes (Von Stockhausen, H.B. ; Biol. Trace Elem. Res. ; 1988; 15; 147-155*).* Une complémentation journalière de 200 µg en sélénium est considérée comme sûre et adéquate pour un homme adulte de poids moyen *(*Schrauzer, G.N., J Am. Col. Nutr. ; 2001; 20 ; 1- 14*).*

Le sélénium se trouve dans la nature, sous deux formes : organique et inorganique.

Les composés inorganiques sont le plus souvent des sels tels que le sélénite ou le sélénate de sodium. Ces composés sont très toxiques pour l'homme et la plupart des animaux.

Les composés organiques (composés organoséléniés) sont représentés dans les organismes vivants notamment par les acides aminés L(+)-sélénométhionine, L(+)-méthylsélénocystéine et L(+)-sélénocystéine.

La L(+)-sélénométhionine est la principale source de sélénium organique chez l'Homme et les animaux. Cependant, l'Homme et les animaux sont autoxotrophes pour cet acide aminé, qui ne peut être obtenu qu'au travers de l'alimentation.

C'est donc sous cette forme organique qu'idéalement le sélénium devrait être incorporé dans les compléments alimentaires visant à traiter ou prévenir une carence en sélénium.

Il a pu être ainsi démontré qu'une supplémentation de l'alimentation en L(+)-sélénométhionine était beaucoup moins toxique et présentait une meilleure biodisponibilité qu'un apport sous forme de sélénite de sodium *(*Mony, MC et al.; J. of Trace Elem. Exp. Med. ; 2000 ; 13 ; 367-380*).*

Actuellement, on ne connaît pas d'autres voies métaboliques de captation du sélénium par les organismes vivants que celles utilisant comme substrats le sélénium inorganique, principalement sous la forme de sélénite de sodium, et la sélénométhionine.

On peut trouver un apport convenable en sélénium organique chez les végétaux supérieurs (blé, maïs, soja notamment), chez lesquels plus de 80% du sélénium est constitué par de la L(+)-sélénométhionine (Schrauzer, G.N. ; J.Am. Coll. Nutrit. ; 2001 ; 20 ; 1 ; 1-4). Cependant la concentration en sélénium de ces végétaux n'est pas suffisante pour pouvoir réaliser facilement, et à moindre coût, des additifs alimentaires.

Une des voies explorées pour obtenir des compositions riches en sélénium, consiste à enrichir certains microorganismes en sélénium organique à partir de sélénium inorganique. Ces microorganismes, une fois enrichis, peuvent servir de matière première à la préparation de produits alimentaires ou cosmétiques.

De nombreuses publications décrivent la préparation de levures enrichies en sélénium, et plus particulièrement la levure *Saccharomyces cerevisiae* (Oh Tae-Kwang et al., brevet KR950006950 du 26.06.1995) en vue de les utiliser en tant que telles ou de les incorporer dans des compositions alimentaires (Moesgaard S. et al., brevet DK200200408 du 16.09.2003) ; ou encore d'obtenir des produits dérivés enrichis en sélénium tels que du pain (Wang Boaquan, brevet CN 1817143 du 16.08.2006), du lait (Jeng Chang-Yi, brevet TW565432 du 11.12.2003), des oeufs (Cui Li *et al.,* brevet CN1302723C du 07.03.2007), du chocolat (In Gyeong Suk et al., brevet KR20040101145 du 08.11.2004) ou de la bière (Jakovleva L.G. et al., brevet RU2209237 du 27.07.2003) enrichis en sélénium. Dans le domaine des aliments de santé, des préparations contenant des levures enrichies en sélénium ont aussi été proposées pour des femmes enceintes (Wang Weiyi, brevet CN1778199 du 31.05.2006), ou encore pour améliorer le micro-environnement intestinal de patients hypoglycémiques (Li Tao Zhao, brevet CN1810161 du 02.08.2006). Dans le domaine dermocosmétique, des compositions contenant des levures enrichies en sélénium ont été développées en vue de réduire la perte capillaire (Kasik Heinz, brevet DE19858670 du 21.06.2000) ou en prévention du photo-vieillissement (Kawai Norihisa et al., brevet JP07300409 du 14.11.1995). Des préparations pharmaceutiques contenant des levures enrichies en sélénium ont été utilisées dans la prévention et le traitement de pathologies inflammatoires telles que les rétinopathies liées au diabète (Crary Ely J., brevet US5639482 du 17.06.1997), ou des pathologies cardiovasculaires (Nagy P.L. *et al.*; brevet HUT060436 du 28.09.1992).

Les bactéries et plus particulièrement les bactéries probiotiques ont, elles aussi, fait l'objet d'enrichissement en sélénium (Calomme M. et al., Biol. Trace Elem. Res.;1995; 47; 379-383*). Lactobacillus acidophilus,* mais aussi *Lactobacillus reuteri, Lactobacillus ferintoshensis, Lactobacillus buchneri*/*parabuchneri* (Andreoni V. et al., brevet US0258964) ont été décrits en tant que compléments alimentaires enrichis en sélénium. Une étude sur *Lactobacillus casei ssp casei* a cependant montré que le sélénium était incorporé majoritairement sous forme de sélénocystéine (Calomme M. et al., Biological Trace Element Research 1995, 47, 379-383). Des mélanges de probiotiques constitués de levures et de lactobacilles, en vue de renforcer le système immunitaire et la résistance aux maladies (Huang Kehe Qin, brevet CN1283171C du 08.11.2006) ont été préparés.

Cependant, dans toutes ces préparations, les microorganismes enrichis en sélénium sont préparés à partir de sélénium inorganique uniquement. Ainsi, la source de sélénium la plus souvent utilisée consiste en du sélénite ou du sélénate de sodium solubilisé dans les milieux de culture des microorganismes. Les microorganismes ainsi enrichis, bien qu'ayant synthétisé des quantités satisfaisantes de sélénium organique assimilable par l'organisme humain, présentent souvent un taux résiduel élevé en sélénium inorganique non transformé, ce qui peut s'avérer dangereux pour le consommateur. De plus, les bactéries, telles que celles du genre *Lactobacillus,* transforment majoritairement ce sélénium inorganique en sélénocystéine et non en sélénométhionine.

Dans une précédente demande publiée sous WO 2006/008190, le demandeur a décrit de nouveaux composés organiques de type séléno-hydroxyacide pouvant servir de précurseurs à la synthèse de L(+)-sélénométhionine chez l'Homme et l'animal.

De manière surprenante, le demandeur a constaté que les composés organiques de type séléno-hydroxyacide décrits dans la demande WO 2006/008190), incorporés dans des milieux de culture, pouvaient être utilisés par différents microorganismes tels que des bactéries et des levures en vue de leur enrichissement en sélénium organique. Les résultats obtenus ont révélé que ces composés permettent d'enrichir très efficacement les microorganismes non photosynthétiques en sélénium organique, et plus particulièrement en sélénométhionine avec un rendement équivalent, voire supérieur, à celui obtenu à l'aide des composés inorganiques habituellement utilisés tel que le sélénite de sodium.

Il est ainsi apparu que l'enrichissement de microorganismes en sélénométhionine à partir de composés organiques de type séléno-hydroxyacide permettait de produire des microorganismes contenant majoritairement la source de sélénium la plus biodisponible pour l'Homme et les animaux et pratiquement exempt de sélénium inorganique. L'invention permet ainsi de résoudre les problèmes de toxicité liés aux procédés de l'art antérieur en diminuant la teneur en sélénium inorganique et de disposer de nouveaux microorganismes enrichis en sélénométhionine.

Les microorganismes non photosynthétiques ainsi enrichis peuvent être directement utilisés dans l'alimentation dans le cadre de la prévention ou du traitement des carences en sélénium, notamment en vue de produire des produits et compositions pharmaceutiques, nutritionnelles ou cosmétiques.

### FIGURES:

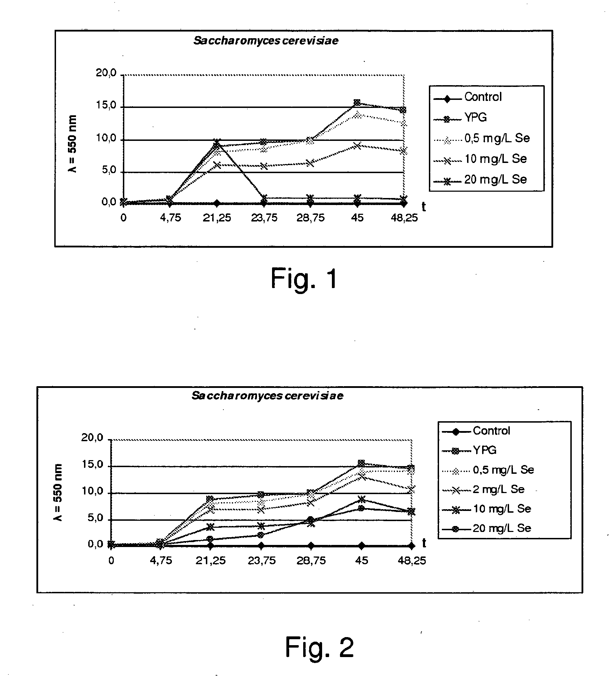

Les figures 1 à 6 ci-après représentent des graphiques montrant l'évolution de la croissance des microorganismes au cours du temps, en milieu YPG (*Saccharomyces cerevisiae*) ou MRS (*Lactobacillus casei*) supplémentés avec différents composés séléniés inorganiques (sélenite de sodium) et organiques (sélénométhionine ou acide 2-hydroxy-4-méthylsélénobutanoïque (THD-177). L'axe des abscisses correspond au nombre d'heures de culture, l'axe des ordonnées à la densité cellulaire (absorbance mesurée à 550 nm).
**Figure 1****:** la levure *Saccharomyces cerevisiae* est cultivée en milieu YPG, supplémentée en sélénite de sodium (composé inorganique)
**Figure 2****:** la levure *Saccharomyces cerevisiae* est cultivée en milieu YPG, supplémentée en sélénométhionine (composé organique)
**Figure 3****:** la levure *Saccharomyces cerevisiae* est cultivée en milieu YPG, supplémentée en acide 2-hydroxy-4-méthylséléno-butanoïque (composé organique selon l'invention)
**Figure 4****:** la bactérie *Lactobacillus casei* est cultivée en milieu MRS, supplémentée en sélénite de sodium (composé inorganique)
**Figure 5****:** la bactérie *Lactobacillus casei* est cultivée en milieu MRS, supplémentée en sélénométhionine (composé organique)
**Figure 6****:** la bactérie *Lactobacillus casei* est cultivée en milieu MRS, supplémentée en acide 2-hydroxy-4-méthylséléno-butanoïque (composé organique selon l'invention)

### Description détaillée de l'invention

La présente demande se limite à l'obtention de microorganismes non-photosynthétiques, c'est-à-dire aux microorganismes dont la croissance ne dépend pas directement d'une source de lumière.

Les résultats expérimentaux obtenus dans le cadre de la présente invention visent plus particulièrement des bactéries non photosynthétiques et des levures, lesquelles ont un métabolisme très différent des microorganismes photosynthétiques.

Par microorganisme, on entend tout organisme vivant unicellulaire appartenant à l'un des Règnes suivants : monères, protistes, mycètes ou protozoaires, présentant une structure cellulaire eucaryote ou procaryote, de taille microscopique ou ultramicroscopique, ayant un potentiel métabolique et de reproduction. Lesdits organismes unicellulaires peuvent être impliqués dans la formation de filaments ou de biofilms.

Par sélénium organique, on entend un ensemble de molécules organiques contenant au moins un composé possédant au moins un atome de sélénium dans sa structure chimique susceptible d'être produit par un organisme vivant, tels que notamment les acides aminés sélénométhionine, méthylsélénocystéine, sélénocystine et sélénocystéine, des peptides ou des protéines les contenant.

De préférence, les microorganismes non photosynthétiques selon l'invention sont des levures ou des bactéries, plus préférentiellement une levure du genre *Saccharomyces* ou une bactérie du genre *Lactobacillus.*

Les microorganismes ainsi enrichis en sélénium organique peuvent être utilisés en tant que tels ou bien en tant qu'additif alimentaire. Ils peuvent, par exemple, être déshydratés pour former une poudre stable pouvant être incorporée dans des compositions servant de base à la préparation de produits transformés mais peuvent être aussi utilisés vivants en tant que probiotiques dans les processus de transformation de produits alimentaires.

La présente invention a donc pour objet un nouveau procédé d'enrichissement de microorganisme non photosynthétique en sélénium organique, caractérisé en ce que ledit microorganisme non photosynthétique est cultivé dans un milieu de culture comprenant un composé de type séléno-hydroxyacide.

De préférence, le composé de type séléno-hydroxyacide est un composé de formule générale (I), un sel, ou bien un dérivé ester ou amide de celui-ci, 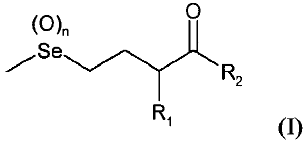 formule dans laquelle :
**n =** 0, 1 ou 2;
**R₁** = OH, OCOR₃, OPO₃H₂, OPO₃R₄R₅ ou OR₆;
**R₂** = OH, R₃, NHR₇, S-cystéinyle ou S-glutathionyle;
   étant entendu que lorsque n = 1 et **R₂** = OH, alors R₁ ne peut être OH ;
**R₃** = alkoxyle, céramide 1, céramide 2, céramide 3, céramide 4, céramide 5, céramide 6a et 6b, S-cystéinyle, S-glutathionyle, ou un groupe choisi parmi les groupes suivants : 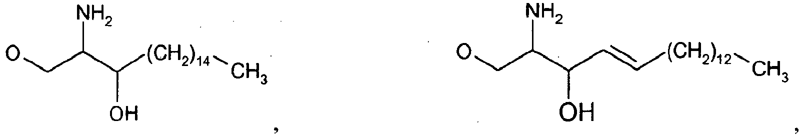 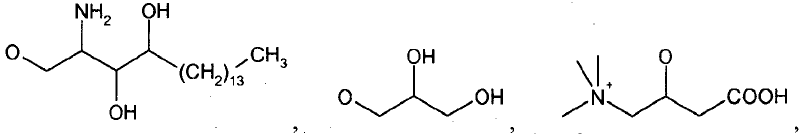 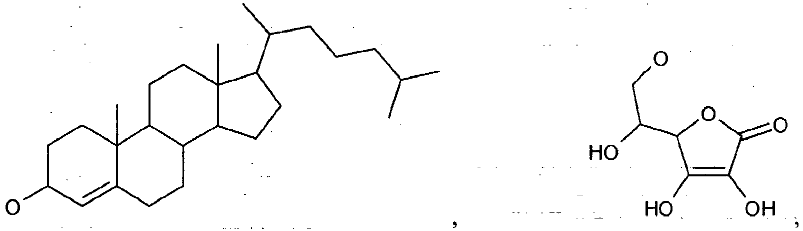 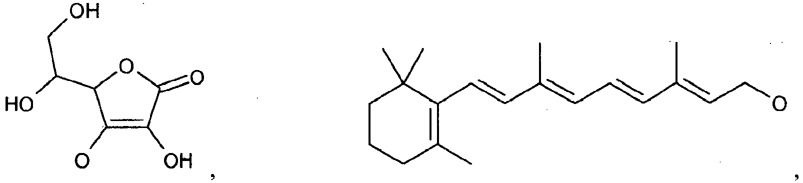 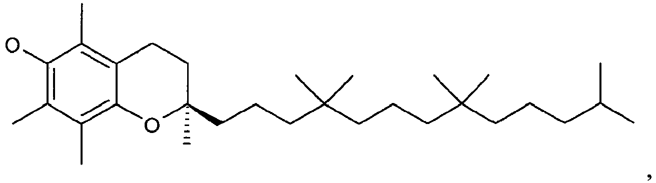 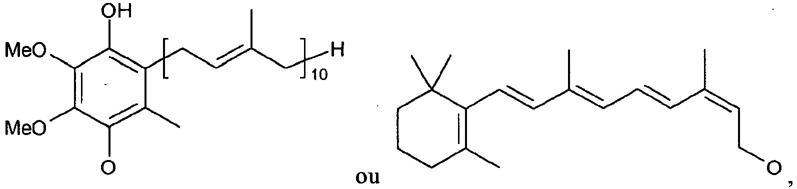
**OR₄** = alkoxyle (C₁-C₂₆), céramide 1, céramide 2, céramide 3, céramide 4, céramide 5, céramide 6a et 6b, ou un groupe choisi parmi les groupes suivants : 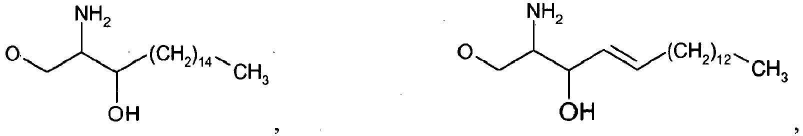 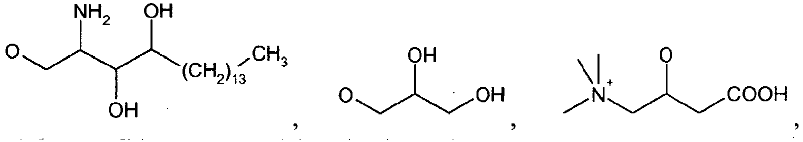 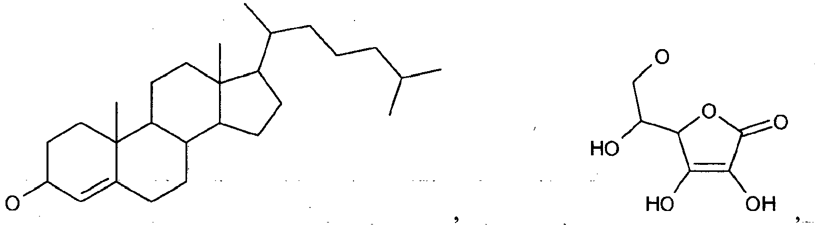 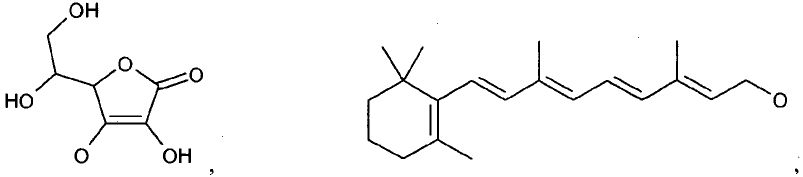 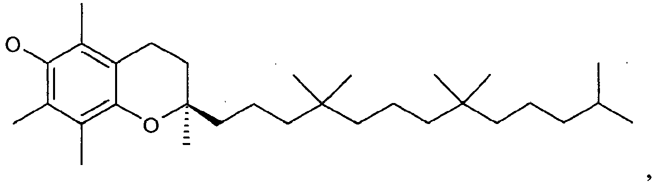 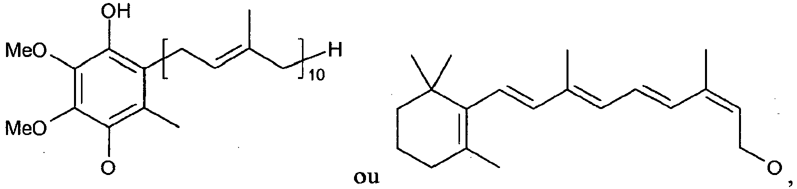
**OR₅** = alkoxyle (C₁-C₂₆), céramide 1, céramide 2, céramide 3, céramide 4, céramide 5, céramide 6a et 6b ou un groupe choisi parmi les groupes suivants : 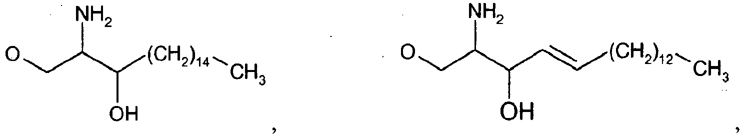 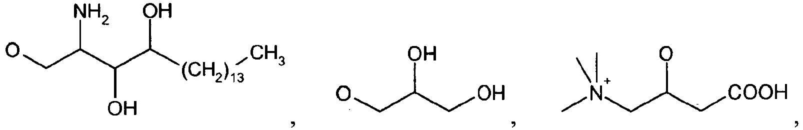 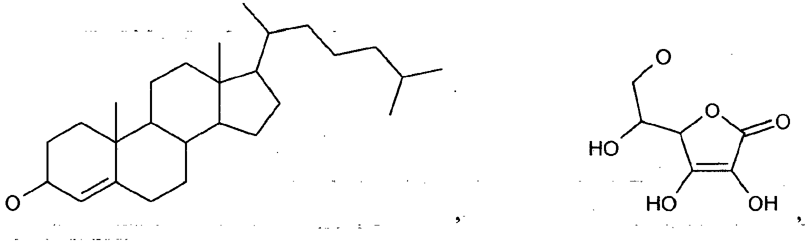 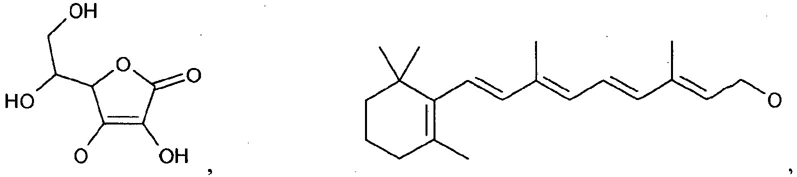 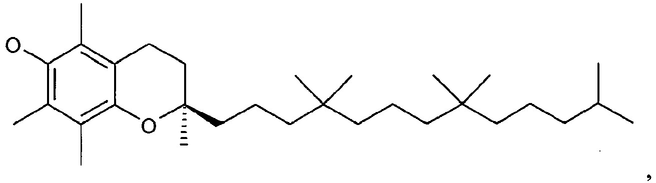 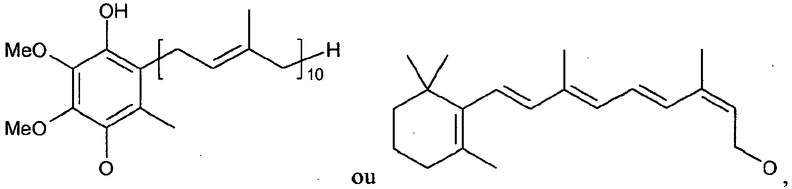
**OR₆** = pyruvate, lactate, citrate, fumarate, maléate, myristate, palmitate, stéarate, palmitoléate, oléate, linoléate, un résidu d'acide gras naturel ou 13-cis rétinoate ;
**NHR₇** = NH₂, NH-alkyle (C₁-C₂₆), un résidu d'amino-acide naturel ou un résidu d'amine naturelle.

Dans la formule (I) ci-dessus :
- par alkyle, il est entendu un groupement comportant 1 à 26, avantageusement 1 à 10, encore avantageusement 1 à 6, atomes de carbone linéaire ou cyclique, éventuellement ramifié, éventuellement fluoré ou polyfluoré, et comportant éventuellement une ou plusieurs doubles liaisons carbone-carbone, tel que par exemple méthyle, éthyle, isopropyle, trifluorométhyle, les chaînes grasses d'acides gras (c'est-à-dire sans la fonction acide) telles que la chaîne grasse de linoléyle, la chaîne grasse de linolényle, la chaîne grasse de palmitoyle.
- par alkoxyle, il est entendu un groupement dérivé d'un alcool primaire, secondaire ou tertiaire, et lié au reste de la molécule par l'intermédiaire de l'atome d'oxygène de la fonction alcool, comportant 1 à 26, avantageusement 1 à 10, encore avantageusement 1 à 6, atomes de carbone linéaire ou cyclique, éventuellement ramifié, éventuellement fluoré ou polyfluoré, et comportant éventuellement une ou plusieurs double liaisons carbone-carbone, tel que par exemple méthoxyle, éthoxyle, isopropoxyle, trifluorométhoxyle.

- des structures de radicaux de type céramide sont décrites notamment dans « Cosmetic Lipids and the Skin Barrier », Thomas Förster Ed. 2002, Marcel Dekker, Inc., page 2, figure 2.
- par naturel, il est entendu tout composé correspondant retrouvé dans le métabolisme des organismes du monde végétal, animal, ainsi que dans celui de l'homme *(*Steglich W., Römpp Encyclopedia Natural Products, G. Thieme ed*.)*
- par acide gras, il est entendu un acide carboxylique aliphatique comprenant de 4 à 28 atomes de carbone (y compris l'atome de carbone de la fonction acide carboxylique), la chaîne hydrocarbonée étant linéaire, saturée ou insaturée.
- par résidu d'acide gras, il est entendu que l'acide gras est lié au reste de la molécule par l'intermédiaire de sa fonction acide carboxylique (COOH).
- par alcool gras, il est entendu un acide gras tel que défini ci-dessus dans lequel la fonction acide carboxylique (COOH) a été remplacé par une fonction alcool (OH).
- par amino-acide naturel, il est entendu notamment les amino-acides suivants : Alanine (Ala), Arginine (Arg), Asparagine (Asn), Acide aspartique (Asp), Cystéine (Cys), Glutamine (Gln), Acide glutamique (Glu), Glycine (Gly), Histidine (His), Isoleucine (Ile), Leucine (Leu), Lysine (Lys), Méthionine (Met), Phénylalanine (Phe), Proline (Pro), Sérine (Ser), Thréonine (Thr), Tryptophane (Trp), Tyrosine (Tyr) et Valine (Val).
- par amine naturelle, il est entendu une amine primaire naturelle portant une fonction NH₂ telle que la putrescine, la cadavérine, la spermine, la spermidine.
- par résidu d'amino-acide ou d'amine, il est entendu que l'amino-acide ou l'amine est lié au reste de la molécule par l'intermédiaire de sa fonction amine primaire (NH₂).
- par oligomère, il est entendu tout composé constitué par l'enchaînement de 2 à 15 monomères reliés entre eux par l'intermédiaire d'une liaison de type ester.
- par polymère, il est entendu tout composé constitué par l'enchaînement de plus de 15 monomères reliés entre eux par l'intermédiaire d'une liaison de type ester.

Selon l'invention, lesdits composés de formule (I) sont préférentiellement utilisés sous forme de sels de calcium, de magnésium, ou de zinc, ce qui permet une meilleure solubilité dans les milieux de culture, ainsi qu'une meilleure assimilation par les microorganismes.

L'invention englobe également les stéréoisomères des composés de formule (I) ainsi que les mélanges de stéréosiomères en toutes proportions et en particulier les mélanges racémiques.

Par « stéréoisomères », on entend, au sens de la présente invention, des diastéréoisomères et des énantiomères. Il s'agit donc d'isomères optiques. Les stéréoisomères qui ne sont pas des images dans un miroir l'un de l'autre sont désignés par « diastéréoisomères », et les stéréoisomères qui sont des images dans un miroir l'un de l'autre, mais non superposables, sont désignés par « énantiomères ».

Un mélange contenant des quantités égales de deux formes énantiomères individuelles de chiralité opposée est désigné par « mélange racémique ».

Avantageusement, n représente 0 dans la formule générale (I).

R₁ peut représenter un groupe OH, OCOR₃ ou OR₆, avec avantageusement R₃ représentant un groupe alkoxyle. En particulier, R₁ pourra représenter un groupe OH.

**R₂** représentera notamment un groupe OH ou R₃, et plus particulièrement un groupe OH ou alkoxyle (C₁-C₂₆).

Les composés séléno-hydroxyacides utilisés dans le cadre de la présente invention pourront répondre plus particulièrement à la formule (Ia) suivante : 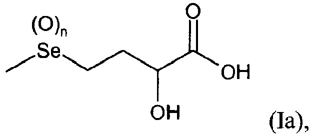 ou à un sel, un stéréoisomère ou un mélange de stéréoisomères en toute proportions, un ester ou un amide de celui-ci,
avec n tel que défini précédemment et de préférence représentant 0.

Par « ester », on entend qu'une fonction ester (-C(O)O-) est formée à partir d'un groupe OH d'une fonction alcool ou acide carboxylique portée par les composés de type séléno-hydroxyacide selon la présente invention. Ces esters sont donc obtenus :
- soit par réaction de la fonction alcool avec un acide carboxylique tel qu'un acide de formule R-COOH avec R = alkyle tel que défmi ci-dessus ; un acide gras naturel ; un amino-acide naturel ; le glutathion ; l'acide pyruvique, lactique, citrique, fumarique, maléique, ou 13-cis-rétinoïque ; notamment avec un acide RCOOH, en particulier avec R représentant un chaîne hydrocarbonée linéaire ou ramifiée, saturée, comportant de 1 à 6 atomes de carbone,
- soit par réaction de la fonction acide carboxylique avec un alcool tel qu'un alcool primaire, secondaire ou tertiaire, de formule R'H avec R' = alkoxyle tel que défini ci-dessus ; un alcool gras ; ou une céramide ; notamment avec un alcool R'H, en particulier avec R représentant un chaîne hydrocarbonée linéaire ou ramifiée, saturée, comportant de 1 à 6 atomes de carbone.

Par « amide », on entend qu'une fonction amide (-C(O)NH-) est formée à partir d'un groupe OH d'une fonction alcool ou acide carboxylique portée par les composés de type séléno-hydroxyacide selon la présente invention. Ces amides sont donc obtenus :
- soit par réaction de la fonction alcool avec un amide dérivé d'un acide carboxylique tel que décrit dans la définition du terme « ester »,
- soit par réaction de la fonction acide carboxylique avec une amine dérivée d'un alcool tel que décrit dans la définition du terme « ester » ou correspondant à un amino-acide naturel ou à une amine naturelle.

L'invention vise plus particulièrement l'utilisation d'un composé de formule (I) choisi parmi :
- l'acide L-2-hydroxy-4-méthylséléno-butanoïque,
- l'acide D-2-hydroxy-4-méthylséléno-butanoïque,
- l'acide D,L-2-hydroxy-4-méthylséléno-butanoïque,
ou un sel de ces composés.

Ces composés sont décrits dans la demande WO 2006/008190.

L'invention a également pour objet un microorganisme, et plus particulièrement une bactérie, non photosynthétique enrichie en sélénium organique, susceptible d'être obtenu selon le procédé de l'invention. Un tel microorganisme présente généralement un contenu en sélénium organique supérieur à 1000 ppm, de préférence supérieur à 1200 ppm, plus préférentiellement supérieur à 1400 ppm, en équivalent sélénium, et un contenu en sélénium inorganique inférieur à 0,5 %, de préférence inférieur à 0,2 % et plus préférentiellement inférieur à 0,1 % en poids sec dudit microorganisme.

L'invention vise également plus particulièrement une levure du genre *Saccharomyces* enrichie en sélénium, caractérisée en ce qu'elle comprend une teneur en sélénométhionine supérieure à 130 microgrammes équivalent sélénium par gramme (µgSe/g), de préférence supérieure à 150 µgSe/g et plus préférentiellement supérieure à 170 µgSe/g en poids sec de ladite levure.

La teneur en sélénium total et en sélénométhionine des microorganismes peut être déterminée respectivement par minéralisation et digestion enzymatique après centrifugation et lyophilisation des microorganismes, par exemple en suivant la méthode selon Lobinsky et *al.* décrite dans Mester, Z. et al. (2006) Annal. Bioanal. Chem. 385 :168-180. La teneur en sélénométhionine correspond à la teneur de la sélénométhionine en tant qu'acide aminé libre et de la sélénométhionine liée à d'autres acides aminés, c'est-à-dire présente dans des protéines et des peptides.

Une levure selon l'invention peut s'avérer utile, par exemple, dans la fabrication de produits de boulangerie enrichis en sélénium mais aussi dans l'obtention de laits d'origine animale enrichis en sélénium organique, ou encore d'oeufs enrichis en sélénium organique.

L'invention vise plus particulièrement une bactérie non photosynthétique probiotiqué, en particulier une bactérie lactique, notamment du genre *Lactobacillus,* caractérisée en ce qu'elle comprend une teneur en sélénométhionine supérieure à 50 µgSe/g, de préférence supérieure à 100 µgSe/g et plus préférentiellement supérieure à 500 µgSe/g en poids sec de ladite bactérie non photosynthétique probiotique.

La sélénométhionine représentera notamment plus de 50%, préférentiellement plus de 60% du sélénium total contenu dans la bactérie.

Une telle bactérie peut s'avérer utile, par exemple, dans la fabrication de laits fermentés, comme des fromages ou des yahourts, enrichis en sélénium organique.

Par « probiotique », on entend un microorganisme qui peut être ingéré par l'animal ou l'homme, sous forme vivante ou morte, et qui présente un effet bénéfique sur l'animal ou l'homme, en termes nutritionnels, pharmaceutiques ou cosmétiques. Dans le cas d'une bactérie, il peut s'agir d'une bactérie lactique, notamment du genre *Lactobacillus.* Les bactéries *Cupriavidus metallidurans* et *Ralstonia metallidurans,* utilisées pour détoxifier les sols, ne répondent pas à cette définition.

Les bactéries probiotiques non photosynthétiques selon la présente invention peuvent donc être utiles à titre de probiotiques, notamment dans un produit cosmétique, pharmaceutique ou nutritionnel.

Les microorganismes non photosynthétiques selon l'invention peuvent ainsi être utiles en nutrition humaine et en nutrition animale, notamment en vue de l'obtention de dérivés secondaires enrichis en sélénium organique, comme par exemple du lait ou des oeufs.

L'invention vise également la fabrication de produits probiotiques, pour une utilisation alimentaire, cosmétique ou pharmaceutique, directement à partir des microorganismes enrichis en sélénium organique selon le procédé de la présente invention. Cette fabrication fait appel aux techniques connues de l'homme du métier.

Selon un mode particulier de l'invention, on incorpore les microorganismes initialement enrichis en sélénium organique selon le procédé de la présente invention, vivants ou morts, en tant qu'additif dans une composition probiotique donnée. Les microorganismes ne participent pas dans ce cas nécessairement à la transformation biologique des ingrédients présents dans la composition.

Selon un autre mode de l'invention, les microorganismes vivants ne sont pas préalablement enrichis en sélénium, mais enrichis au fur et à mesure du procédé de fabrication de la composition à partir d'un ou plusieurs composés de formule (I) tels que définis plus haut, selon le procédé de l'invention. Lesdits composés de formule (I) sont alors incorporés dans ladite composition avec les autres ingrédients et microorganismes non photosynthétiques. Les microorganismes non photosynthétiques peuvent alors opérer une bioconversion des composés de sélénium organique à l'intérieur même de la composition pour obtenir, par exemple, une pâte à pain dont les levures sont enrichies en sélénium organique ou encore un lait fermenté dans lequel les bactéries lactiques sont enrichies en sélénium organique.

Dans ces compositions, on utilisera plus particulièrement une bactérie probiotique telle que définie précédemment, par exemple une bactérie lactique, notamment du genre *Lactobacillus.*

Le sélénium organique et produits dérivés ainsi obtenus sont utiles dans différentes applications, dont celles rappelées en préambule, notamment en tant qu'agent cosmétique, pharmaceutique ou nutritionnel.

L'invention vise également les compositions probiotiques (à usage cosmétique, pharmaceutique ou nutritionnelle) comprenant un ou plusieurs microorganismes enrichis en sélénium organique selon l'invention, et plus particulièrement une bactérie probiotique telle que définie précédemment, par exemple une bactérie lactique, notamment du genre *Lactobacillus.*

Par composition ou produit probiotique, on entend une composition ou un produit comprenant un microorganisme, vivant ou mort, utilisé à titre de probiotique tel que défini ci-dessus.

L'invention vise encore un milieu de culture pour microorganisme non photosynthétique caractérisé en ce qu'il comprend un ou plusieurs des composés de formule (I) définis plus haut. Un tel milieu de culture est utile pour la mise en oeuvre du procédé d'enrichissement des microorganismes en sélénium organique selon l'invention. En particulier, l'invention vise un milieu de culture comprenant au moins un composé de formule (I) tel que défini précédemment, de préférence l'acide 2-hydroxy-4-méthylsélénobutanoïque ou l'un de ses sels, ou l'un de ses esters ou amides ou de ses stéréoisomères ou mélanges de stéréoisomères à une concentration comprise entre 0,5 et 2000 mg/L, de préférence entre 1 et 1000 mg/L, plus préférentiellement entre 2 et 500 mg/L, soit respectivement environ entre 0,2 et 800 mg/L dudit composé en équivalent sélenium, préférentiellement entre 0,4 et 400 mg/L dudit composé en équivalent sélenium, plus préférentiellement entre 0,8 et 200 mg/L dudit composé en équivalent sélenium.

Un procédé de préparation de microorganisme non photosynthétique selon l'invention peut notamment comprendre une ou plusieurs des étapes suivantes :
- préparer un milieu de culture, de préférence un milieu minimum, comportant les éléments chimiques nécessaires à la croissance dudit microorganisme non photosynthétique;
- introduire dans le milieu de culture un composé de formule (I), préférentiellement de l'acide 2-hydroxy-4méthylséléno-butanoïque ou l'un de ses sels en tant que source organique de sélénium;
- ajuster le pH du mélange à une valeur comprise entre 3 et 8;
- mettre en culture un inoculum de pré-culture dudit microorganisme non photosynthétique dans le mélange ainsi constitué, à une température comprise entre 25 et 80°C, sous une agitation orbitale comprise entre 100 et 500 rpm, et une atmosphère pouvant contenir de 0 à 20% en oxygène et de 0,5 à 99 % en gaz carbonique, de préférence pendant 24 à 96 heures;
- centrifuger le mélange entre 4000 et 10 000 rpm pendant quelques minutes;
- reprendre le culot cellulaire dans de l'eau physiologique;
- centrifuger à nouveau entre 4000 et 10 000 rpm pendant quelques minutes;
- stériliser le culot cellulaire humide dans lequel se trouve le microorganisme non photosynthétique enrichi en sélénium ;

Le culot cellulaire humide peut être lyophilisé ou séché à l'air.

Le milieu de culture peut être notamment un milieu synthétique ou semi-synthétique, un milieu enrichi, ou un milieu sélectif.

Après centrifugation et lyophilisation des microorganismes, les teneurs en sélénium total et en sélénométhionine peuvent être déterminées, par exemple, après respectivement minéralisation et digestion enzymatique par la méthode selon Lobinsky, R. et al décrite dans Mester, Z. et al. (2006) Anal. Bioanal. Chem. 385 :168-180.

D'autres caractéristiques et avantages de l'invention sont donnés dans les exemples qui suivent. Les exemples ci-après sont fournis uniquement à titre d'illustration et ne sauraient en aucune façon limiter la portée de l'invention.

### EXEMPLES

### Exemple 1: Production de la levure Saccharomyces cerevisiae dans un milieu YPG contenant de l'acide 2-hydroxy-4-méthylséléno-butanoïque et dans un milieu YPG contenant du sélénite de sodium:

### • Isolement de la souche de Saccharomyces cerevisiae 3053-E000

La souche a été isolée à partir d'un carré de 42g de levure de panification de la marque FALA BACKHEFE, d'un lot identifié avec une DLC 22/07/2007. Cette levure a été conservée au réfrigérateur à +4°C avant utilisation, puis elle a été reprise en milieu liquide YPG (Yeast Peptone Glucose). La souche de *Saccharomyces cerevisiae:* 3053-E000 a été obtenue par étalement sur milieu YPG en colonies isolées et mise en collection à -80°C avec ajout de 10% vol/vol de Glycérol comme agent cryoprotecteur.

| *Composition du milieu YPG :* | |
|---|---|
| Yeast Extract | 10 g |
| Peptone | 20 g |
| Glucose | 20 g |
| pH | 7.0 |
| Eau osmosée, qsp | 1 L |

Dans les expérimentations décrites les caractéristiques de croissance de la levure *Saccharomyces cerevisiae* 3053 E000 ont été mesurées en présence de différentes concentrations de sélénium sous la forme de sélénite de Sodium, de sélénométhionine, ou d'acide 2-hydroxy-4-méthylséléno-butanoïque et comparées aux caractéristiques de croissance en l'absence d'additifs (contrôle). La pré-culture de la souche *Saccharomyces cerevisiae* 3053-E000 est réalisée en milieu liquide YPG, à 37°C, sous agitation orbitale (250 rpm) pour une durée de 24h.

### • Conditions de culture de la souche Saccharomyces cerevisiae 3053-E000

La culture est réalisée à partir d'un inoculum (1.10⁶ UFC/ml) de la pré-culture précédemment décrite dans 100 mL de milieu YPG, à pH = 4 à une température de 37°C sous agitation (250 rpm) et selon les conditions suivantes :
Les cultures de *Saccharomyces cerevisiae* 3053-E000 ont été suivies par des mesures d'absorbance à 550 nm et par des comptages CFU sur boites gélosés YPG.

### • Addition de sélénium sous la forme de sélénite de sodium

Les concentrations testées pour l'ajout de sélénite de sélénium sont de 0,5 mg/L, 10 mg/L et 20 mg/L en équivalent sélénium, soit respectivement : 1,1 mg/L, 22,22 mg/L et 44,44 mg/L de sélénite de sodium.

Le graphique de la figure 1 permet de visualiser la croissance de la souche de *Saccharomyces cerevisiae* 3053-E000 en présence des différentes concentrations de sélénite de sodium. Comme on peut l'observer, le sélénite de sodium commence à manifester un effet toxique à partir de 10 mg/L (en équivalent sélénium), affectant négativement la vitesse de croissance et le rendement obtenu en biomasse (- 50% en 48h).

A une concentration de 20 mg/L (en équivalent sélénium), le sélénite de sodium se révèle très toxique pour *Saccharomyces cerevisiae.*

### • Addition de sélénium sous la forme de sélénométhionine

Les concentrations testées pour l'ajout de sélénométhionine sont de 0,5 mg/L, 2 mg/L, 10 mg/L et 20 mg/L en équivalent sélénium, soit respectivement 1,25 mg/L, 5 mg/L, 25 mg/L et 50 mg/L de sélénométhionine

Le graphique de la figure 2 permet de visualiser la croissance de la souche de *Saccharomyces cerevisiae* 3053-E000 en présence des différentes concentrations de sélénométhionine. Comme on peut l'observer, la sélénométhionine commence à manifester un effet à partir de 2 mg/L (en équivalent sélénium) affectant négativement le rendement obtenu en biomasse (- 29% en 48h). L'augmentation de la dose à 10 mg/L (en équivalent sélénium) produit des effets inhibiteurs plus importants sur la vitesse de croissance et le rendement biomasse (- 50%) sans affecter pour autant la viabilité (- 30%). Pour 20 mg/L de sélénométhionine (en équivalent sélénium), les effets sont similaires à ceux obtenus pour 10mg/L en ce qui concerne la vitesse et le rendement final en biomasse, l'effet toxique sur la viabilité se manifestant plus fortement (- 50%).

### • Addition de sélénium sous la forme d'acide 2-hydroxy-4-méthylséléno-butanoïque

Les concentrations testées pour l'ajout d'acide 2-hydroxy-4-méthylséléno-butanoïque sont de 0,5 mg/L, 2 mg/L, 10 mg/L et 20 mg/L en équivalent sélénium, soit respectivement 1,25 mg/L, mg/L, 25 mg/L et 50 mg/L d'acide 2-hydroxy-4-méthylséléno-butanoïque (THD-177, Tetrahedron, France, CAS :873660-49-2).

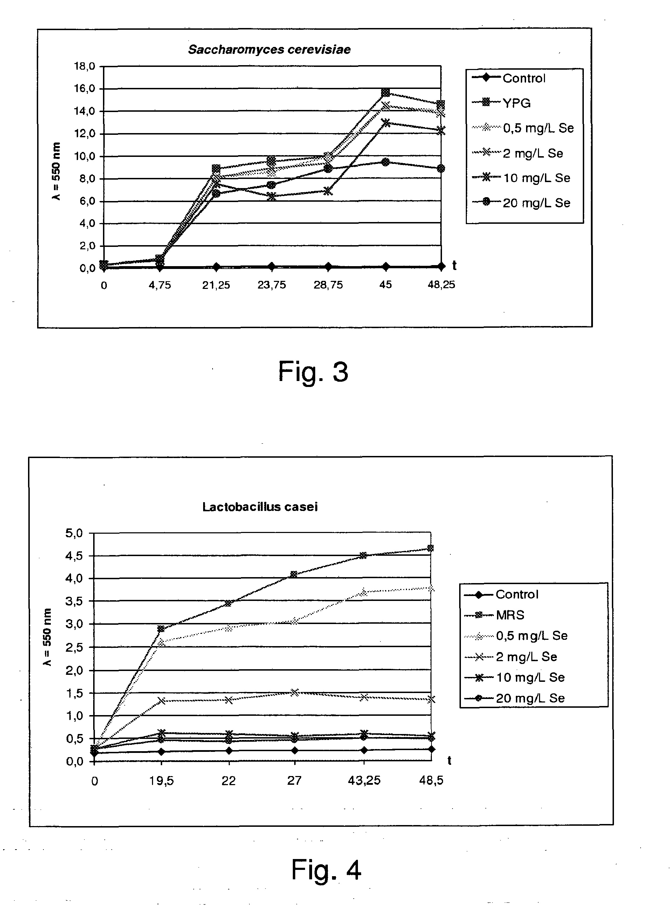

Le graphique de la figure 3 permet de visualiser la croissance de la souche de *Saccharomyces cerevisiae* 3053-E000 en présence des différentes concentrations d'acide 2-hydroxy-4-méthylséléno-butanoïque. Comme on peut l'observer, l'acide 2-hydroxy-4-méthylséléno-butanoïque commence à manifester un effet à partir de 10 mg/L (en équivalent sélénium) affectant négativement le rendement final en biomasse (-15%) et en réduisant la viabilité de 36%. Pour une concentration de 20 mg/L d'acide 2-hydroxy-4-méthylséléno-butanoïque (en équivalent sélénium), le rendement en biomasse est réduit de 36% et la viabilité est réduite de 50%.

### • Enrichissement en sélénium de Saccharomyces cerevisiae

Au vu des résultats obtenus précédemment pour la production de biomasse de la souche de *Saccharomyces cerevisiae* 3053-E000, il a été procédé au choix d'un taux d'incorporation de 20 mg/L d'acide 2-hydroxy-4-méthylséléno-butanoïque (en équivalent sélénium) et d'un taux d'incorporation de 10 mg/L de sélénite de sodium (en équivalent sélénium) dans les cultures décrites ci-après :
Pré-cultures : à partir d'une culture saturée en YPG, il a été effectué deux étapes de pré-culture.
Pré-culture 1 : 10 mL de culture (taux inoculation 10% v/v à partir de la culture saturée, incubation 24h à 37°C)
Pré-culture 2 : 100 mL de culture (taux inoculation 10% v/v à partir de la préculture 1, incubation 24h à 37°C)
Culture : à partir de la pré-culture 2, 1 L de milieu YPG est inoculé à un taux de 10% v/v. La culture est incubée à une température fixée et régulée à 37°C. Le pH est ajusté à 4 et la culture est mélangée par agitation orbitale (150 rpm).

### Préparation des échantillons pour analyses :

Après 48 heures de culture, le milieu est centrifugé à 6500 rpm pendant 5 minutes, le culot cellulaire est repris dans de l'eau physiologique puis centrifugé à nouveau à 6500 rpm pendant 5 minutes.

Le culot cellulaire humide est lyophilisé pour l'analyse des constituants séléniés (sélénium total, sélénométhionine et sélénite de sodium).

### • Analyse des constituants séléniés de la levure Saccharomyces cerevisiae produite dans un milieu contenant de l'acide 2-hydroxy-4-méthylséléno-butanoïque ou du sélénite de sodium

Le sélénium total est dosé par ICP couplée à une détection par masse, après minéralisation de l'échantillon. La spéciation du sélénium est effectuée par chromatographie liquide haute performance couplée à une détection masse-masse, après digestion enzymatique de l'échantillon (Mester, Z. et al. (2006) Annal. Bioanal. Chem. 385 :168-180).

### • Résultats

Les concentrations en sélénium total, en sélénométhionine et en sélénite de sodium sont données dans le tableau suivant :

**Tableau 1**

| Analyse des composants séléniés de la levure *Saccharomyces cerevisiae* 3053-E000 produite sur milieu YPG avec additifs | | | | |
|---|---|---|---|---|
| | Sélénium total^{a} | Sélénométhionine^{a} totale | Selenométhionine/ Sélénium total | Sélénite^{a} |
| Témoin | 0,17 ± 0,02 | 0,053 ± 0,006 | - | <0,02 |
| + Sélénite (10 mgSe/L) | 849 ± 12 | 122 ± 1 | 14% | 14 ± 1 |
| + THD 177 (20 mgSe/L) | 601 ± 15 | 185 ± 21 | 31% | 3,3 ± 0,1 |

| | | | | |
|---|---|---|---|---|
| a : µg (équivalent Se)/g | | | | |

Les résultats obtenus reportés dans le tableau 1 montrent que l'incorporation d'acide 2-hydroxy-4-méthylséléno-butanoïque dans le milieu de culture permet d'enrichir par un facteur d'environ 3500 la teneur totale en sélénium de *Saccharomyces cerevisiae,* la sélénométhionine constituant 31% de ce sélénium total. Le sélénite de sodium permet d'avoir un facteur d'enrichissement supérieur, de l'ordre de 5000, mais la sélénométhionine ne représente que 14% de la teneur totale en sélénium.

Par ailleurs, la teneur résiduelle en sélénite, dans la levure *Saccharomyces cerevisiae,* liée à l'utilisation d'acide 2-hydroxy-4-méthylséléno-butanoïque comme source de sélénium, est plus de 4 fois inférieure à celle correspondant à l'utilisation du sélénite de sodium comme source de sélénium.

L'acide 2-hydroxy-4-méthylséléno-butanoïque constitue donc bien une meilleure source de sélénium que le sélénite de sodium pour enrichir en sélénométhionine un microorganisme eucaryote tel que *Saccharomyces cerevisiae,* tout en diminuant de 400% le risque de toxicité liée à la teneur résiduelle en sélénite dans le microorganisme.

### Exemple 2: Production de la bactérie Lactobacillus paracasei dans un milieu MRS contenant de l'acide 2-hydroxy-4-méthylséléno-butanoïque et dans un milieu MRS contenant du sélénite de sodium

### • Isolement de la souche de Lactobacillus paracasei 3052 E000

La souche a été isolée à partir d'un tube d'ACTIMEL^{®} commercialisé par la société Danone.

Ce tube a été conservé au réfrigérateur à +4°C avant utilisation, puis son contenu a été dilué dans 50 mL de milieu liquide MRS.

La souche de *Lactobacillus paracasei* 3052-E000 a été obtenue par étalement sur milieu MRS en colonies isolées. Elle a été caractérisée sur Galerie API 50 CHL et mise en collection à -80°C avec ajout de 10% vol/vol de glycérol comme agent cryoprotecteur.

| *Composition et préparation pour 1L de milieu MRS :* | |
|---|---|
| Polypeptone | 10g |
| Extrait de levure | 5g |
| Extrait de viande | 10g |
| Glucose | 20g |
| Phosphate dipotassique | 2g |
| Acétate de sodium | 5g |
| Citrate d'ammonium | 2g |
| Sulfate de magnésium | 0,20g |
| Sulfate de manganèse | 0,05g |
| Tween 80 | 1ml |
| pH | 6,4 |

Dans cette expérimentation, les caractéristiques de croissance de la bactérie *Lactobacillus paracasei* ont été mesurées en présence de différentes concentrations de sélénium sous la forme de sélénite de sodium, de sélénométhionine, de l'acide 2-hydroxy-4-méthylséléno-butanoïque et comparées aux caractéristiques de croissance en l'absence d'additifs.

### • Pré-culture de la souche de Lactobacillus paracasei 3052 E000

La pré-culture de la souche *Lactobacillus paracasei* 3052-E000 est réalisée en milieu liquide MRS, à 42°C, sans agitation pour une durée de 24h.

La culture de la souche *Lactobacillus paracasei* 3052-E000 a été réalisée à partir d'un inoculum (1.10⁷ UFC/ml) de la préculture ci-dessus dans 100 mL de milieu MRS, à pH = 6,4 et à une température de 42°C (sans agitation).

Les cultures ont été suivies par des mesures en absorbance à 550 nm et sur comptage CFU réalisé sur boite MRS.

### • Addition de sélénium sous la forme de sélénite de sodium

Les concentrations testées pour l'ajout de sélénite sélénium sont de 0,5 mg/L, 2 mg/L, 10 mg/L et 20 mg/L en équivalent sélénium, soit respectivement 1,11 mg/L, 4,44 mg/L, 22,22 mg/L et 44,44 mg/L de sélénite de sodium .Le graphique de la figure 4 permet de visualiser la croissance de la souche de *Lactobacillus paracasei* 3052-E000 en présence de sélénite de sodium.

Comme on peut l'observer, le sélénite de sodium commence à manifester un effet inhibiteur sur la croissance de *Lactobacillus paracasei* dès l'addition de 0,5 mg /L de sélénite de sodium (en équivalent sélénium), ainsi qu'une réduction de rendement de production de biomasse de 25% à 48h. Ces effets sont accentués avec l'addition de 2 mg/L de sélénite de sodium (en équivalent sélénium), pour atteindre une réduction de rendement atteignant 90% à 48h pour des concentrations de 10 et 20 mg/L de sélénite de sélénium (en équivalent sélénium). A ces niveaux, le sélénite de sodium manifeste une toxicité sur *Lactobacillus paracasei.*

### • Addition de sélénium sous la forme de sélénométhionine

Les concentrations testées pour l'ajout de sélénométhionine sont de 0,5 mg/L, 2 mg/L, 10 mg/L et 20 mg/L en équivalent sélénium, soit respectivement 1,25 mg/L, 5 mg/L, 25 mg/L et 50 mg/L de sélénométhionine.

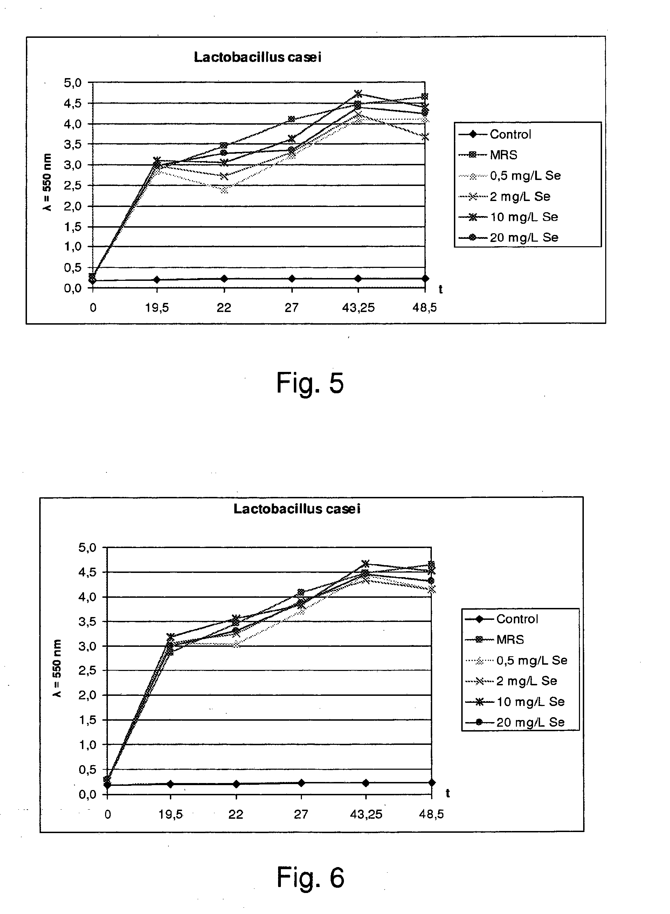

Le graphique de la figure 5 permet de visualiser la croissance de la souche de *Lactobacillus paracasei* 3052-E000 en présence des différentes concentrations de sélénométhionine.

L'addition de sélénium sous la forme de sélénométhionine ne montre pas d'effet inhibiteur sur la croissance, ni d'effet sensible de réduction du rendement de production de la biomasse jusqu'à 20 mg/L (en équivalent sélénium) sur *Lactobacillus paracasei.*

### • Addition de sélénium sous la forme d'acide 2-hydroxy-4-méthylséléno-butanoïque

Les concentrations testées pour l'ajout d'acide 2-hydroxy-4-méthylséléno-butanoïque sont de 0,5 mg/L, 2 mg/L, 10 mg/L et 20 mg/L en équivalent sélénium, soit respectivement 1,25 mg/L, 5 mg/L, 25 mg/L et 50 mg/L d'acide 2-hydroxy-4-méthylséléno-butanoïque (THD-177, Tetrahedron, France, CAS :873660-49-2).

Le graphique de la figure 6 permet de visualiser la croissance de la souche de *Lactobacillus paracasei* 3052-E000 en présence des différentes concentrations d'acide 2-hydroxy-4-méthylséléno-butanoïque.

De manière similaire à l'addition de sélénométhionine, l'addition de sélénium sous la forme d'acide 2-hydroxy-4-méthylséléno-butanoïque ne montre pas d'effet inhibiteur sur la croissance ni d'effet sensible de réduction du rendement de production de la biomasse jusqu'à 20 mg/L d'acide 2-hydroxy-4-méthylséléno-butanoïque (en équivalent sélénium).

Afin de compléter l'étude d'impact de l'ajout de l'acide 2-hydroxy-4-méthylséléno-butanoïque, à des valeurs plus importantes, des concentrations de 40 et de 60 mg/L ont été testées dans les mêmes conditions que précédemment. De même que pour l'addition de 20 mg/L de sélénium sous forme d'acide 2-hydroxy-4-méthylséléno-butanoïque, aucun effet inhibiteur n'a été constaté sur la croissance et le rendement biomasse de la souche.

### • Enrichissement en sélénium de Lactobacillus paracasei

Au vu des résultats obtenus précédemment, il a été procédé au choix d'un taux d'incorporation de 60 mg/L d'acide 2-hydroxy-4-méthylséléno-butanoïque (en équivalent sélénium) et d'un taux d'incorporation de 1 mg/L de sélénite de sélénium (en équivalent sélénium) pour la production de biomasse de la souche de *Lactobacillus paracasei* 3052-E000,

Préparation des échantillons pour analyses :
Après 48 heures de culture, le milieu est centrifugé à 6500 rpm pendant 5 minutes, le culot cellulaire est repris dans de l'eau physiologique puis centrifugé à nouveau à 6500 rpm pendant 5 minutes.

### • Analyse des constituants séléniés de la bactérie Lactobacillus paracasei produite dans un milieu contenant de l'acide 2-hydroxy-4-méthylséléno-butanoïque ou du sélénite de sodium

Le sélénium total est dosé par ICP couplée à une détection par masse, après minéralisation de l'échantillon. La spéciation du sélénium est effectuée par chromatographie liquide haute performance couplée à une détection masse-masse, après digestion enzymatique de l'échantillon.

### • Résultats

Les concentrations en sélénium total, en sélénométhionine et en sélénite de sodium sont données dans le tableau suivant :

**Tableau 2**

| Analyse des composants séléniés de la bactérie *Lactobacillus paracasei* 3052-E000 produite sur milieu MRS avec additifs | | | | |
|---|---|---|---|---|
| | Sélénium total^{a} | Sélénométhionine^{a} totale | Selenométhionine/ Sélénium total | Sélénite^{a} |
| Témoin | 0,18 ± 0,02 | 0,108 ± 0,006 | - | <0,02 |
| +Sélénite (1 mgSe/L) | 120 ± 2 | 17 ± 2 | 14% | <0,5 |
| +THD 177 (60 mg/L) | 1019 ± 19 | 671 ± 117 | 66% | <0,5 |

| | | | | |
|---|---|---|---|---|
| a : µg (équivalent Se)/g | | | | |

Les résultats obtenus dans le tableau 2 montrent que l'incorporation d'acide 2-hydroxy-4-méthylséléno-butanoïque dans le milieu de culture permet d'enrichir par un facteur d'environ 5700 la teneur en sélénium total de *Lactobacillus paracasei,* la sélénométhionine constituant 66% de ce sélénium total. Le facteur d'enrichissement en sélénium total suite à l'incorporation de sélénite de sodium dans le milieu de culture est très inférieur et n'est que de 700, et la sélénométhionine ne représentant que 14% de la teneur totale en sélénium. L'acide 2-hydroxy-4-méthylséléno-butanoïque constitue donc bien une meilleure source de sélénium que le sélénite de sodium pour enrichir en sélénométhionine un microorganisme procaryote tel que *Lactobacillus paracasei.*

### Exemple 3 : Production de la bactérie Lactobaccilus plantarum 3120-E000 dans un milieu MRS contenant de l'acide 2-hydroxy-4-méthylséléno-butanoïque après 47h de culture

Dans cet exemple, les conditions de culture sont identiques à celles de l'exemple 2. Les résultats correspondants de ces travaux sont décrits dans le tableau suivant :

**Tableau 3**

| Analyse des composants séléniés de la bactérie *Lactobacillus plantarum* 3120-E000 produite sur milieu MRS avec additifs | | | |
|---|---|---|---|
| | Sélénium total^{a} | Sélénométhionine^{a} totale | Sélénite^{a} |
| Témoin | 0,289 ± 0,014 | 0,26 ± 0,03 | < 0,2 |
| +THD 177 (10 mg/L) | 582 ± 14 | 512 ± 35 | 0,27 ± 0, 03 |
| +THD 177 (60 mg/L) | 2015 ± 78 | 1813 ± 152 | 1,4 ± 0,4 |

| | | | |
|---|---|---|---|
| a : µg (équivalent Se)/g | | | |

Les résultats obtenus dans le tableau 3 montrent que l'incorporation d'acide 2-hydroxy-4-méthylséléno-butanoïque dans le milieu de culture, à 10mg/L ou à 60mg/L, pendant 47h permet d'enrichir en sélénium total par un facteur d'environ 2000 ou 7000 respectivement. Ces résultats montrent en outre que la sélénométhionine constitue plus de 88% du sélénium total.

### Exemple 4 : Production de la bactérie Escherichia coli WT 3121-E000 dans un milieu minimum (M63 + glucose + méthionine) contenant de l'acide 2-hydroxy-4-méthylséléno-butanoïque aprés 48h de culture

L'origine de la souche *Escherichia coli* WT 3121-E000 est la suivante : *Escherichia coli* K12 (souche 58), numéro de référence CGSC 5587

| *Composition et préparation pour 1L de milieu de culture :* | |
|---|---|
| KH₂PO₄ | 13,6 g |
| KOH | 4,2 g |
| (NH₄)₂SO₄ | 2,0 g |
| FeSO₄, 7H₂O | 1,08 mg |
| Thiamine | 1 mg |
| MgSO₄, 7H₂O | 246 mg |
| Glucose | 4,0 g |
| Méthionine | 50,0 mg |
| pH | 7,0 |
| Eau osmosée, qsp | 1 L |

Les résultats correspondants de ces travaux sont décrits dans le tableau suivant :

**Tableau 4**

| Analyse des composants séléniés de la bactérie *Escherichia coli* WT 3121-E000 produite sur milieu minimum (M63 + glucose + méthionine) avec additifs | | | |
|---|---|---|---|
| total^{a} | Sélénium total^{a} | Sélénométhionine^{a} totale | Sélénite^{a} |
| Témoin | 0,164 ± 0,140 | 0,140 ± 0,018 | <0,1 |
| +THD 177 (60 mg/L) | 6310 ± 78 | 6223 ± 432 | 2,1 ± 0,4 |

| | | | |
|---|---|---|---|
| a : µg (équivalent Se)/g | | | |

Les résultats obtenus dans le tableau 4 montrent que l'incorporation d'acide 2-hydroxy-4-méthylséléno-butanoïque dans le milieu de culture, à 60mg/L, pendant 48h permet d'enrichir en sélénium total par un facteur de plus de 38 000. Ces résultats montrent en outre que la sélénométhionine constitue plus de 98% du sélénium total.

## Revendications

1. Procédé d'enrichissement d'un microorganisme non photosynthétique en sélénium organique **caractérisé en ce que** ledit microorganisme non photosynthétique est cultivé dans un milieu comprenant un composé de type séléno-hydroxyacide.

2. Procédé selon la revendication 1, **caractérisé en ce que** le composé de type séléno-hydroxyacide est de formule générale (I) telle que définie ci-après, un sel, un stéréoisomère, un mélange de sétérosiomères en toutes proportions tel qu'un mélange racémique, ou un dérivé ester ou amide dudit composé 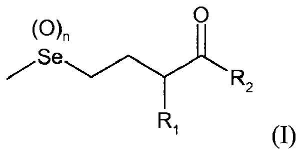 formule dans laquelle :
n = 0, 1 ou 2;
**R₁** = OH, OCOR₃, OPO₃H₂, OPO₃R₄R₅ ou OR₆;
**R₂** = OH, R₃, NHR₇, S-cystéinyle ou S-glutathionyle;
étant entendu que lorsque n = 1 et R₂ = OH, alors R₁ ne peut être OH ;
**R₃** = alkoxyle, céramide 1, céramide 2, céramide 3, céramide 4, céramide 5, céramide 6a et 6b, S-cystéinyle, S-glutathionyle, ou un groupe choisi parmi les suivants : 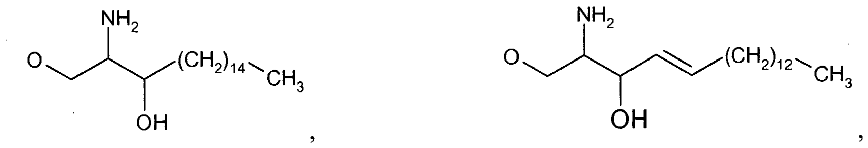 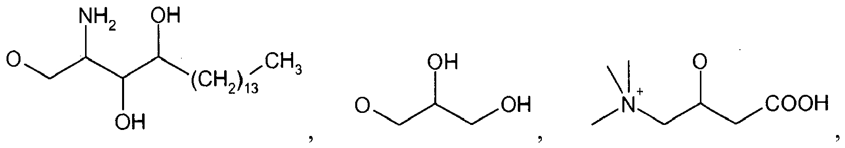 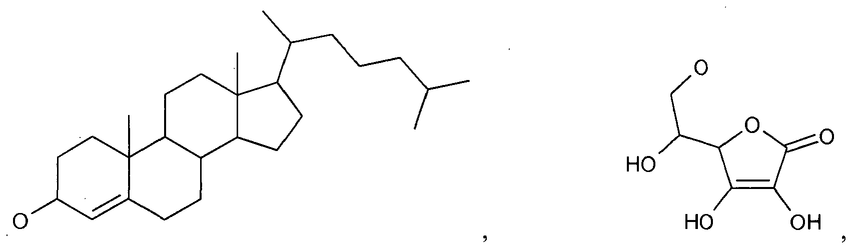 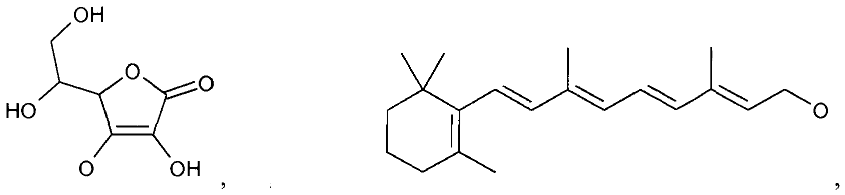 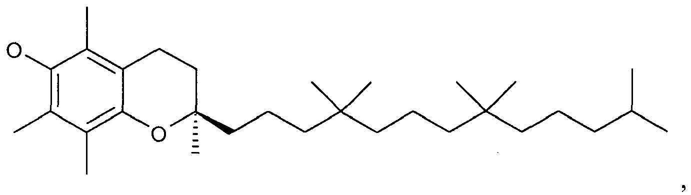 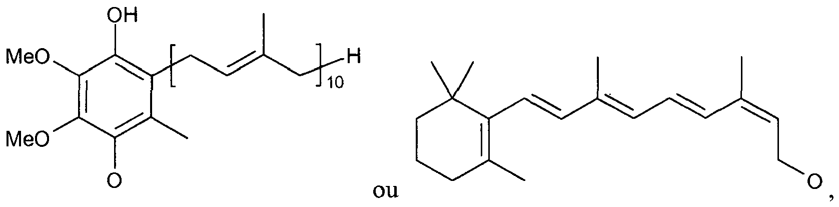
**OR₄** = alkoxyle (C₁-C₂₆), céramide 1, céramide 2, céramide 3, céramide 4, céramide 5, céramide 6a et 6b, ou un groupe choisi parmi les suivants : 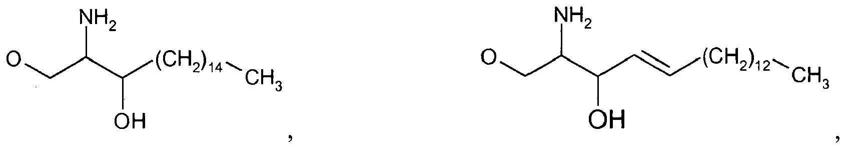 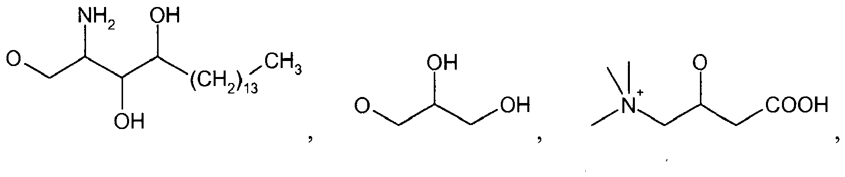 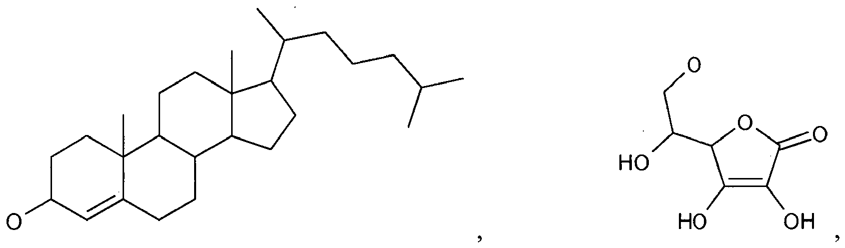 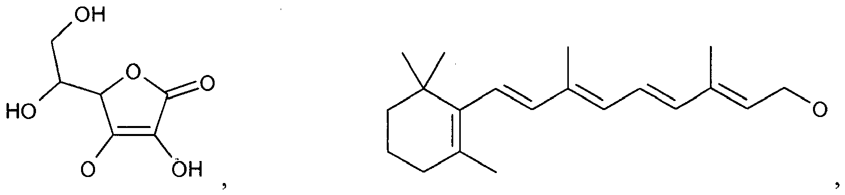 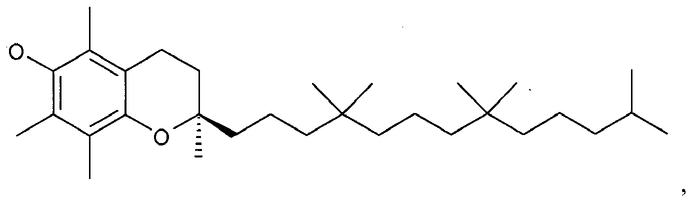 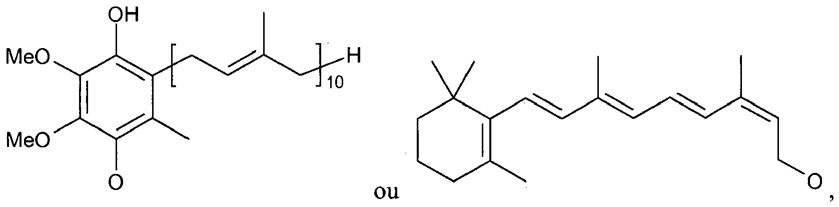
**OR₅** = alkoxyle (C₁-C₂₆), céramide 1, céramide 2, céramide 3, céramide 4, céramide 5, céramide 6a et 6b ou un groupe choisi parmi les suivants : 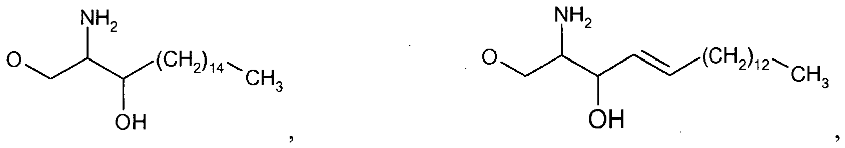 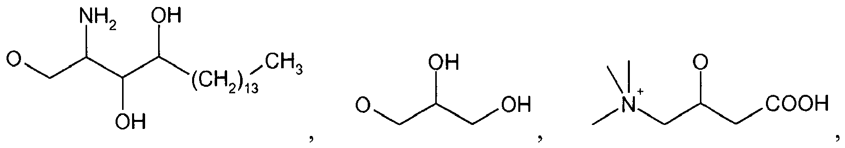 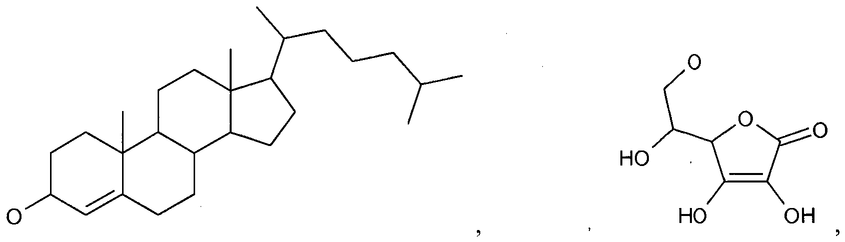 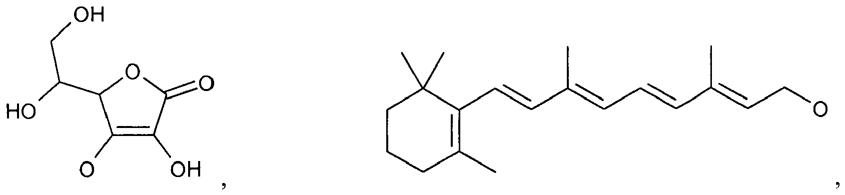 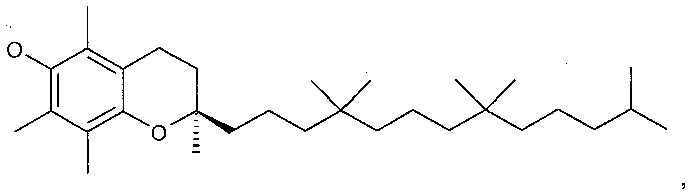 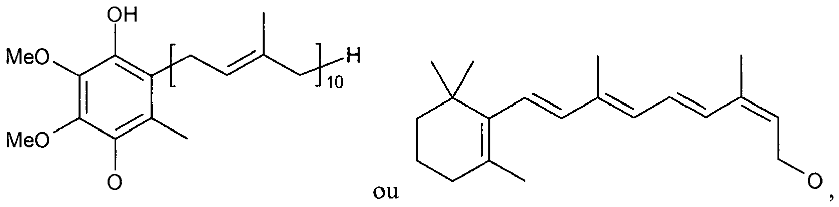
**OR₆** = pyruvate, lactate, citrate, fumarate, maléate, myristate, palmitate, stéarate, palmitoléate, oléate, linoléate, un résidu d'acide gras naturel ou 13-cis rétinoate ;
**NHR₇** = NH₂, NH-alkyle (C₁-C₂₆), un résidu d'amino-acide naturel ou un résidu d'amine naturelle.

3. Procédé selon la revendication 1 ou 2, **caractérisé en ce que** ledit composé de type séléno-hydroxyacide répond à la formule (Ia) suivante : 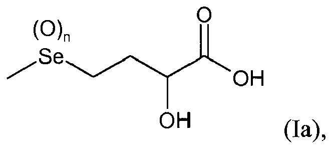 ou à un sel, un stéréoisomère ou un mélange de sétérosiomères en toutes proportions tel qu'un mélange racémique, un ester ou un amide de celui-ci, avec n tel que défini précédemment et de préférence représentant 0.

4. Procédé selon l'une quelconque des revendications 1 à 3, **caractérisé en ce que** ledit composé de type séléno-hydroxyacide est choisi parmi :
- l'acide L-2-hydroxy-4-méthylséléno-butanoïque,
- l'acide D-2-hydroxy-4- méthylséléno-butanoïque,
- l'acide D,L-2-hydroxy-4- méthylséléno-butanoïque,
ou l'un de ses sels.

5. Procédé selon l'une quelconque des revendications 1 à 4, **caractérisé en ce que** ledit composé de type séléno-hydroxyacide est sous forme de sels de calcium, de zinc ou de magnésium.

6. Procédé selon l'une quelconque des revendications 1 à 5, **caractérisé en ce que** le microorganisme non photosynthétique est sélectionné parmi une levure non photsynthétique, telle qu'une levure du genre *Saccharomyces,* ou une bactérie non photosynthétique, telle qu'une bactérie lactique, notamment du genre *Lactobacillus.*

7. Bactérie probiotique non-photosynthétique enrichie en sélénium organique, **caractérisée en ce qu'**elle comprend une teneur en sélénométhionine supérieure à 50 µgSe/g, de préférence supérieure à 100 µgSe/g et plus préférentiellement supérieure à 500 µgSe/g en poids sec de ladite bactérie probiotique non-photosynthétique, et **caractérisée en ce que** la sélénométhionine représente plus de 50% du sélénium total contenu dans la bactérie.

8. Bactérie probiotique non photosynthétique selon la revendication 7, **caractérisée en ce qu'**il s'agit d'une bactérie lactique, notamment du genre *Lactobacillus.*

9. Bactérie probiotique non photosynthétique selon la revendication 7 ou 8, **caractérisée en ce qu'**elle comprend, en outre, moins de 0,5 %, préférentiellement moins de 0,2 %, et plus préférentiellement moins de 0,1 % en poids sec de sélénium inorganique.

10. Bactérie probiotique non photosynthétique selon l'une quelconque des revendications 7 à 9, pour son utilisation à titre de probiotique.

11. Composition probiotique comprenant une bactérie probiotique non photosynthétique selon l'une quelconque des revendications 7 à 9, vivante ou morte.

12. Composition probiotique selon la revendication 11 pour son utilisation en tant que composition cosmétique ou nutritionnelle.

13. Milieu de culture pour microorganisme non photosynthétique **caractérisé en ce qu'**il comprend un composé de type séléno-hydroxyacide tel que défini dans l'une quelconque des revendications 1 à 5.

## Patentansprüche

1. Verfahren zum Anreichern eines nicht photosynthetischen Mikroorganismus mit organischem Selen, **dadurch gekennzeichnet, dass** der nicht photosynthetische Mikroorganismus in einem Medium kultiviert wird, das eine Selenohydroxysäure-Verbindung enthält.

2. Verfahren nach Anspruch 1, **dadurch gekennzeichnet, dass** die Selenohydroxysäure-Verbindung die nachstehend definierte allgemeine Formel (I) aufweist, ein Salz, ein Stereoisomer, eine Mischung aus Stereoisomeren in jedem Verhältnis wie ein Racemat oder ein Ester- oder Amid-Derivat der Verbindung: 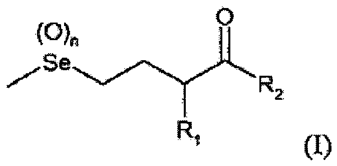 ist, wobei in der Formel:
n = 0, 1 oder 2;
R₁ = OH, OCOR₃, OPO₃H₂, OPO₃R₄R₅ oder OR₆;
R₂ = OH, R₃, NHR₇, S-Cysteinyl oder S-Glutathionyl;
wobei wenn n = 1 und R₂ = OH, R₁ nicht OH sein kann;
R₃ = Alkoxyl, Ceramid 1, Ceramid 2, Ceramid 3, Ceramid 4, Ceramid 5, Ceramid 6a und 6b, S-Cysteinyl, S-Glutathionyl oder eine Gruppe, die aus Folgendem ausgewählt wird: 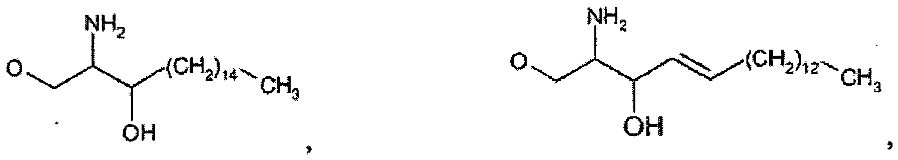 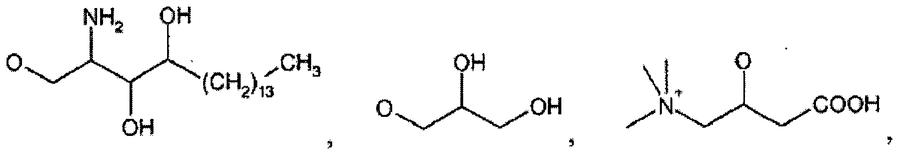 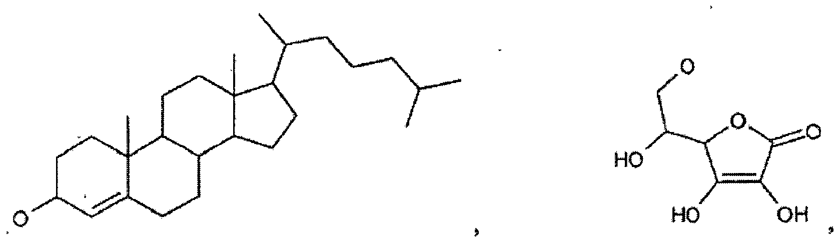 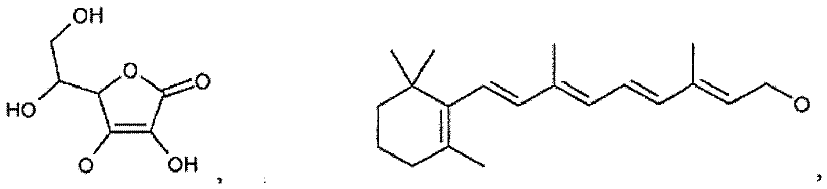 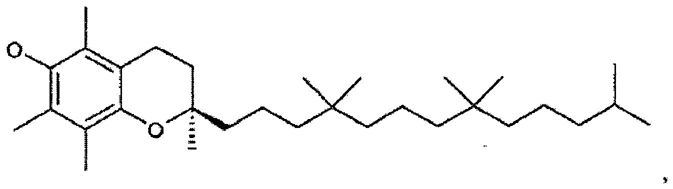 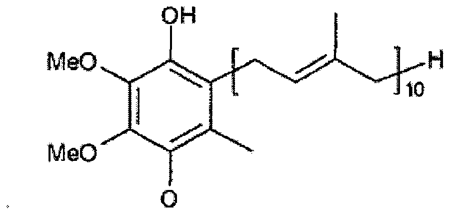 oder 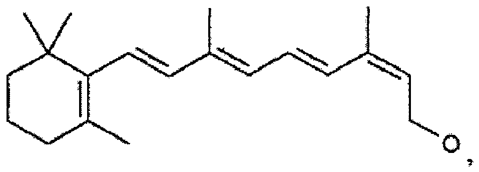
OR₄ = Alkoxyl (C₁-C₂₆), Ceramid 1, Ceramid 2, Ceramid 3, Ceramid 4, Ceramid 5, Ceramid 6a und 6b oder eine Gruppe, die aus Folgendem ausgewählt wird: 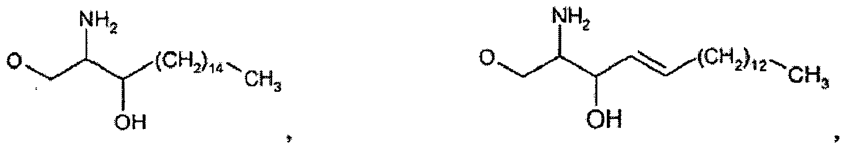 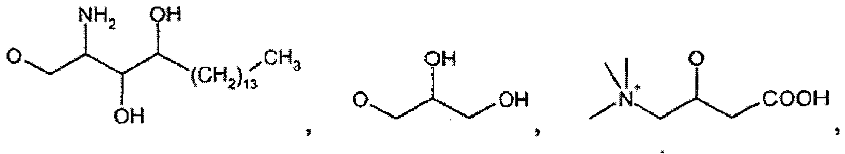 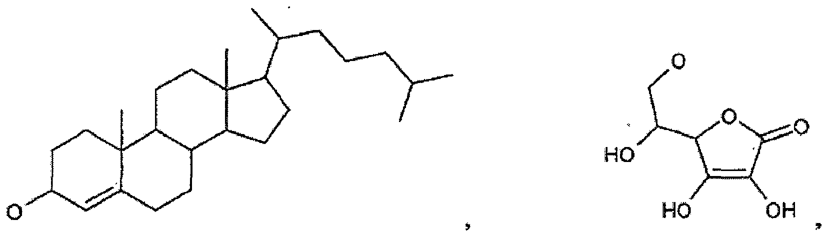 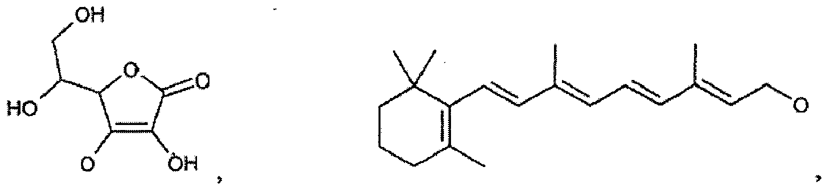 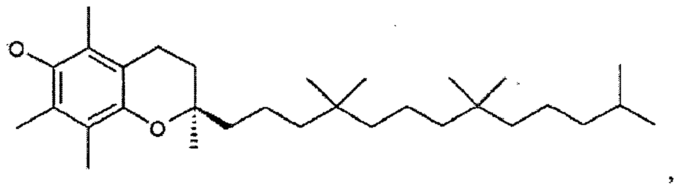 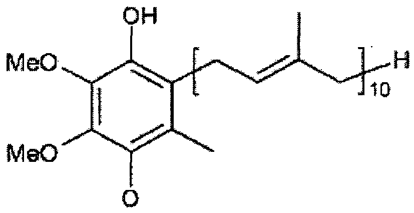 oder 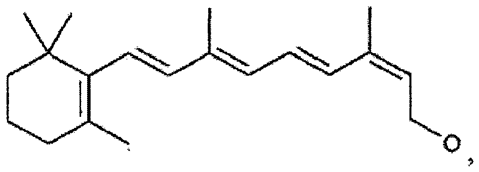
OR₅ = Alkoxyl (C₁-C₂₆), Ceramid 1, Ceramid 2, Ceramid 3, Ceramid 4, Ceramid 5, Ceramid 6a und 6b oder eine Gruppe, die aus Folgendem ausgewählt wird: 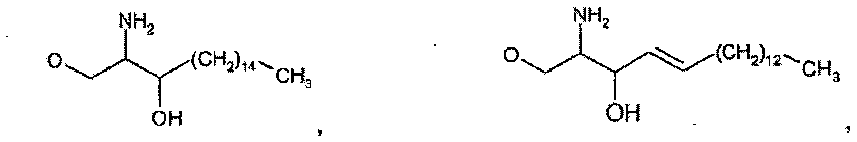 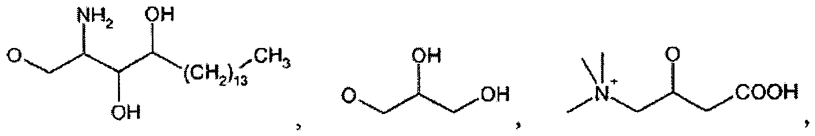 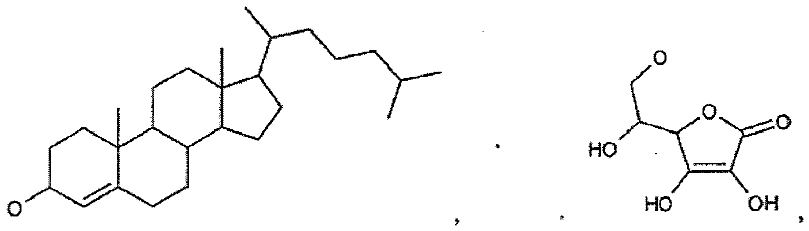 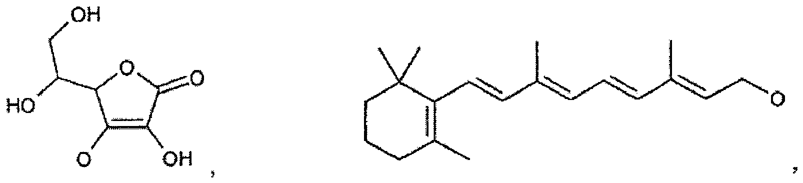 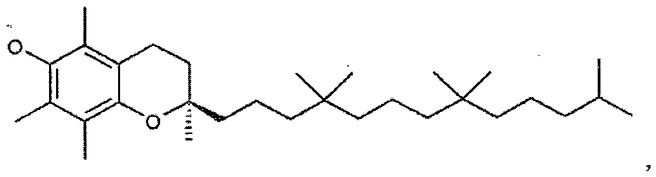 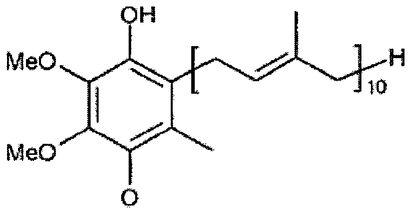 oder 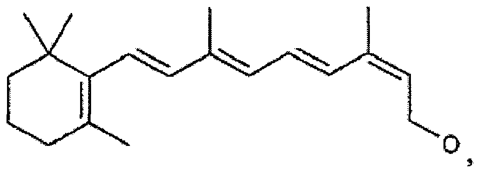
OR₆ = Pyruvat, Lactat, Citrat, Fumarat, Maleat, Myristat, Palmitat, Stearat, Palmitoleat, Oleat, Linoleat, ein Rest einer natürlichen Fettsäure oder 13-cis-Retinoat;
**NHR₇** = NH₂, NH-Alkyl (C₁-C₂₆), ein Rest einer natürlichen Aminosäure oder ein Rest eines natürlichen Amins.

3. Verfahren nach Anspruch 1 oder 2, **dadurch gekennzeichnet, dass** die Selenohydroxysäure-Verbindung folgender Formel (la) entspricht: 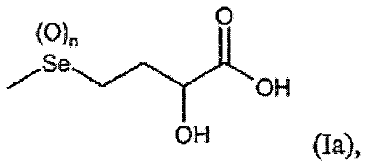 oder einem Salz, einem Stereoisomer oder einer Mischung aus Stereoisomeren in jedem Verhältnis wie einem Racemat, einem Ester oder einem Amid derselben, wobei n wie zuvor definiert lautet und vorzugsweise 0 ist.

4. Verfahren nach einem der Ansprüche 1 bis 3, **dadurch gekennzeichnet, dass** die Selenohydroxysäure-Verbindung aus Folgendem ausgewählt wird:
- L-2-Hydroxy-4-methylseleno-butansäure,
- D-2-Hydroxy-4-methylseleno-butansäure,
- D,L-2-Hydroxy-4-methylseleno-butansäure,
oder einem ihrer Salze.

5. Verfahren nach einem der Ansprüche 1 bis 4, **dadurch gekennzeichnet, dass** die Selenohydroxysäure-Verbindung in Form von Calcium-, Zink- oder Magnesiumsalzen vorliegt.

6. Verfahren nach einem der Ansprüche 1 bis 5, **dadurch gekennzeichnet, dass** der nicht photosynthetische Mikroorganismus aus einer nicht photosynthetischen Hefe wie einer Hefe der Gattung *Saccharomyces* oder einem nicht photosynthetischen Bakterium wie einem Milchsäurebakterium, insbesondere der Gattung *Lactobacillus,* ausgewählt wird.

7. Mit organischem Selen angereichertes, nicht photosynthetisches probiotisches Bakterium, **dadurch gekennzeichnet, dass** es einen Selenomethionin-Gehalt von über 50 µg Se/g, vorzugsweise über 100 µg Se/g und bevorzugter über 500 µg Se/g im Trockengewicht des nicht photosynthetischen probiotischen Bakteriums aufweist, und **dadurch gekennzeichnet, dass** das Selenomethionin mehr als 50 % des in dem Bakterium enthaltenen Gesamtselens ausmacht.

8. Nicht photosynthetisches probiotisches Bakterium nach Anspruch 7, **dadurch gekennzeichnet, dass** es sich um ein Milchsäurebakterium, insbesondere der Gattung *Lactobacillus,* handelt.

9. Nicht photosynthetisches probiotisches Bakterium nach Anspruch 7 oder 8, **dadurch gekennzeichnet, dass** es ferner weniger als 0,5 %, vorzugsweise weniger als 0,2 % und bevorzugter weniger als 0,1 % anorganisches Selen im Trockengewicht enthält.

10. Nicht photosynthetisches probiotisches Bakterium nach einem der Ansprüche 7 bis 9, für seine Verwendung als Probiotikum.

11. Probiotische Zusammensetzung, die ein lebendes oder totes, nicht photosynthetisches probiotisches Bakterium nach einem der Ansprüche 7 bis 9 enthält.

12. Probiotische Zusammensetzung nach Anspruch 11 für ihre Verwendung als Zusammensetzung für die Kosmetik oder die Ernährung.

13. Kulturmedium für einen nicht photosynthetischen Mikroorganismus, **dadurch gekennzeichnet, dass** es eine in einem der Ansprüche 1 bis 5 definierte Selenohydroxysäure-Verbindung enthält.

## Claims

1. A method of enriching a non-photosynthetic micro-organism with organic selenium **characterised in that** said non-photosynthetic micro-organism is cultivated in a medium comprising a compound of seleno-hydroxyacid type.

2. The method according to claim 1, **characterised in that** the compound of seleno-hydroxyacid type is of general formula (I) as defined hereafter, a salt, a stereoisomer, a mixture of stereoisomers in all proportions such as a racemic mixture, or an ester or amide derivative of said compound 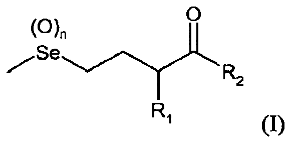 formula in which:
n = 0, 1 or 2;
R₁ = OH , OCOR₃, OPO₃H₂ , OPO₃R₄R₅ or OR₆ ;
R₂ - OH, R₃ , NHR₇, S-cysteinyl or S-glutathionyl ;
it being understood that when n = 1 and R₂ = OH, then R₁ cannot be OH;
R₃ = alkoxyl, ceramide 1, ceramide 2, ceramide 3, ceramide 4, ceramide 5, ceramide 6a and 6b, S-cysteinyl, S-glutathionyl, or a group chosen from the following: 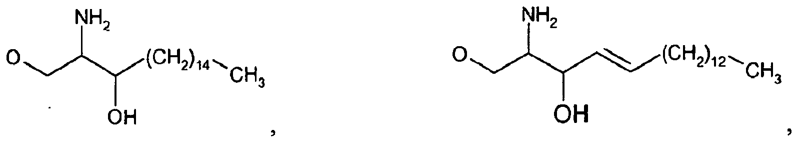 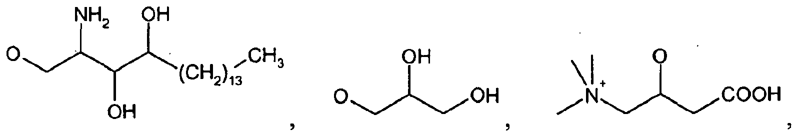 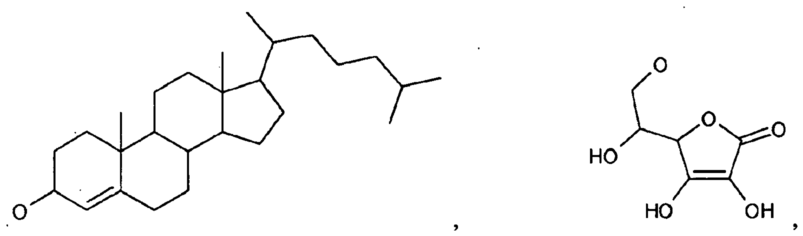 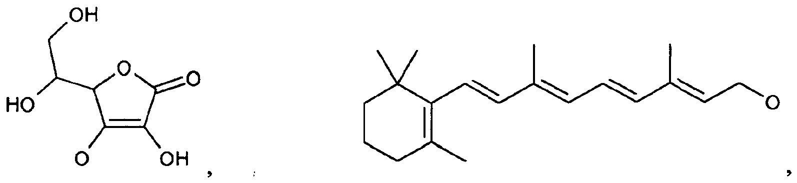 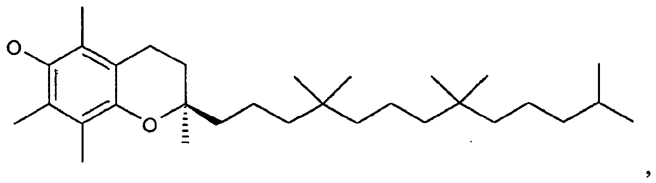 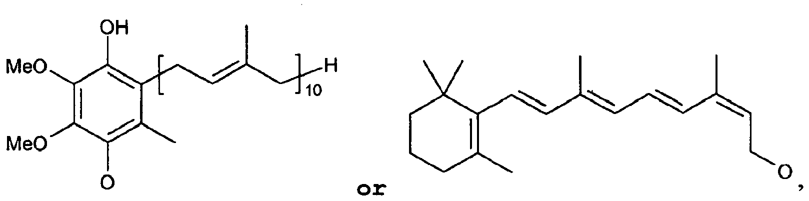
OR₄ = alkoxyl (C₁-C₂₆), ceramide 1, ceramide 2, ceramide 3, ceramide 4, ceramide 5, ceramide 6a and 6b or a group chosen from the following: 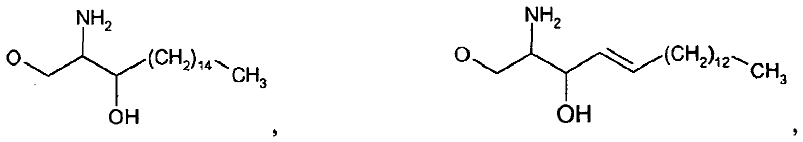 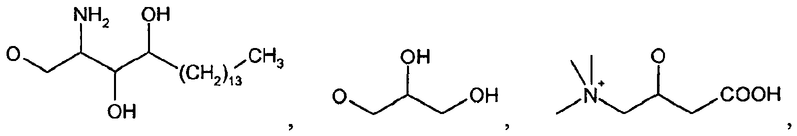 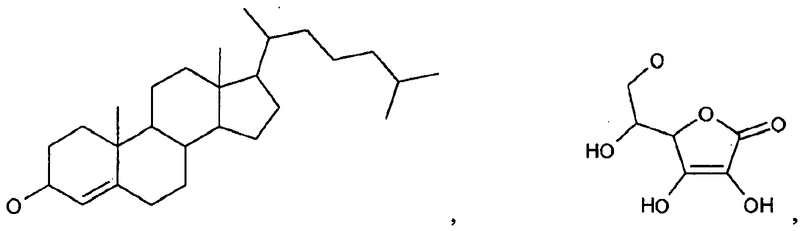 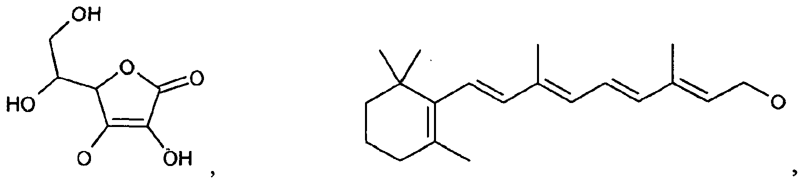 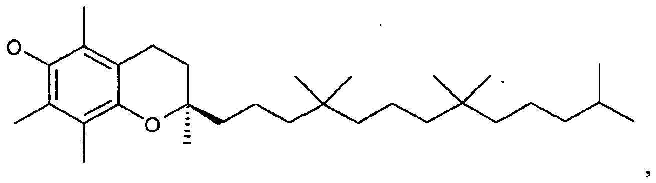 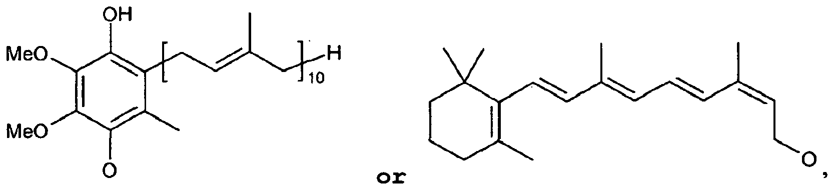
OR₅ = alkoxyl (C₁-C₂₆), ceramide 1, ceramide 2, ceramide 3, ceramide 4, ceramide 5, ceramide 6a and 6b or a group chosen from the following: 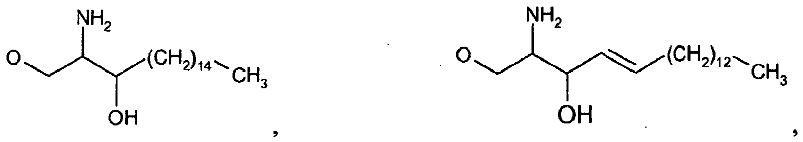 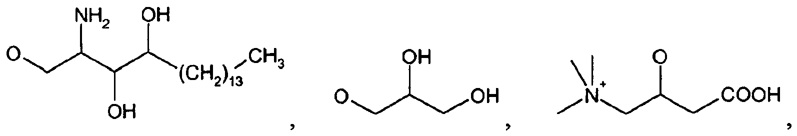 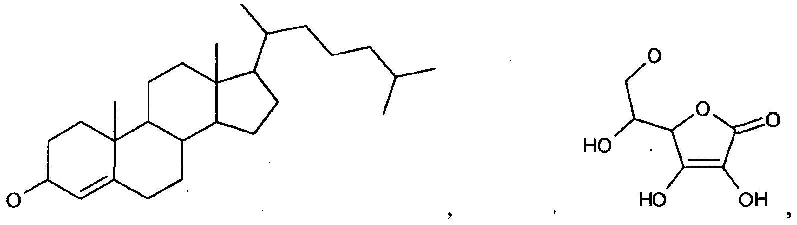 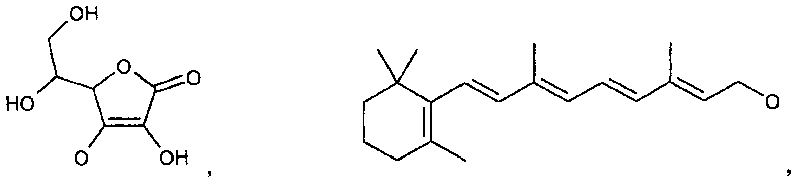 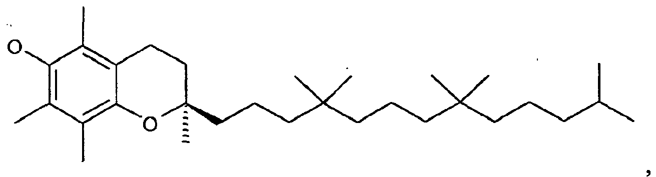 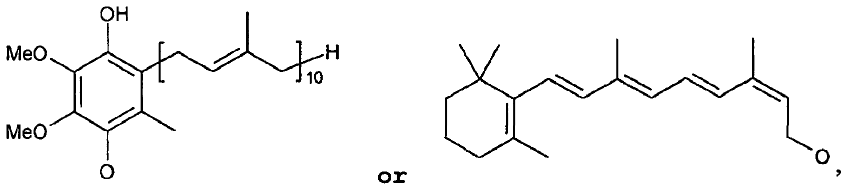
OR₆ = pyruvate, lactate, citrate, fumarate, maleate, myristate, palmitate, stearate, palmitoleate, oleate, linoleate, a residue of natural fatty acid or 13-cis retinoate;
**NHR₇** = NH₂, NH-alkyl (C₁-C₂₆), a residue of natural amino acid or a residue of natural amine.

3. The method according to claim 1 or 2, **characterized in that** said compound of seleno-hydroxyacid type corresponds to the following formula (Ia) : 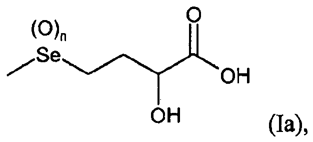 or a salt, a stereoisomer or a mixture of stereoisomers in all proportions such as a racemic mixture, an ester or an amide thereof, with n as defined previously and preferably representing 0.

4. The method according to any of claims 1 to 3, **characterised in that** said compound of seleno-hydroxyacid type is chosen from:
- L-2-hydroxy-4-methylseleno-butanoic acid,
- D-2-hydroxy-4-methylseleno-butanoic acid,
- D,L-2-hydroxy-4-methylseleno-butanoic acid,
or a salt thereof.

5. The method according to any of claims 1 to 4, **characterised in that** said compound of seleno-hydroxyacid type is in the form of calcium, zinc or magnesium salts.

6. The method according to any of claims 1 to 5, **characterised in that** the non-photosynthetic micro-organism is selected from a non-photosynthetic yeast, such as a yeast of the genus *Saccharomyces,* or a non-photosynthetic bacterium, such as a lactic bacterium, especially of the genus *Lactobacillus.*

7. A non-photosynthetic probiotic bacterium enriched with organic selenium, **characterised in that** it comprises a selenomethionine content greater than 50 µgSe/g, preferably greater than 100 µgSe/g and more preferentially greater than 500 µgSe/g in dry weight of said non-photosynthetic probiotic bacterium, and **characterised in that** the selenomethionine represents more than 50% of the total selenium contained in the bacterium.

8. The non-photosynthetic probiotic bacterium according to claim 7, **characterised in that** it is a lactic bacterium, especially of the genus *L**actobacillus.***

9. The non-photosynthetic probiotic bacterium according to claim 7 or 8, **characterised in that** it comprises, moreover, less than 0.5 %, preferentially less than 0.2 %, and more preferentially less than 0.1 % in dry weight of inorganic selenium.

10. The non-photosynthetic probiotic bacterium according to any of claims 7 to 9, for its use as probiotic.

11. A probiotic composition comprising a non-photosynthetic probiotic bacterium according to any of claims 7 to 9, living or dead.

12. The probiotic composition according to claim 11 for its use as cosmetic or nutritional composition.

13. A culture medium for non-photosynthetic micro-organism **characterised in that** it comprises a compound of seleno-hydroxyacid type as defined in any of claims 1 to 5.
